# EUROPEAN PATENT APPLICATION

(11) **EP 2 404 566 A1**
(43) Date of publication of application: **11.01.2012**
(21) Application number: 10748681.3
(22) Date of filing: 26.02.2010
(51) Int. Cl.: A61B 19/00, A61B 17/16, B23B 39/14, B23Q 17/24

(54) **NAVIGATION SYSTEM FOR REMOTELY OPERATED ACTUATOR**

(30) Priority: 06.03.2009 JP 2009053263; 06.03.2009 JP 2009053264
(71) Applicant: NTN Corporation, Osaka-shi, Osaka 550-0003 (JP)
(72) Inventor: NISHIO, Yukihiro, Iwata-shi Shizuoka 438-0037 (JP); NAGANO, Yoshitaka, Iwata-shi Shizuoka 438-0038 (JP)
(74) Representative: Hirsch & Associés
(86) International application number: PCT/JP2010/053088
(87) International publication number: WO 2010/101086

(57) **Abstract**

A remote controlled actuator (5) includes an actuator main body (10), a distal end member (2) fitted to a distal end of a spindle guide section (3) of the actuator main body (10) for alteration in attitude, and a tool (1) rotatably supported by the distal end member (2). The navigation system includes a tool processing member position estimator (55) for estimating the position of a processing member (1a) of the tool (1) from information on the position and attitude of a marker (7A), fitted to the actuator main body (10), that are detected by a marker detecting unit (8), information on the attitude of the distal end member (2) relative to the actuator main body (10), information on the relative position of the distal end member (2) relative to the marker (7A), and information on the shape of the tool (1).

## Description

### CROSS REFERENCE TO THE RELATED APPLICATION

This application is based on and claims Convention priority to Japanese patent applications No. 2009-053263 and No. 2009-053264, both filed March 6, 2009, the entire disclosures of which are herein incorporated by reference as a part of this application.

### BACKGROUND OF THE INVENTION

### (Field of the Invention)

The present invention relates to a navigation system for a remote controlled actuator of a kind, which is used in medical and mechanical processing applications and capable of altering the attitude of a machine tool by remote control.

### (Description of Related Art)

Remote controlled actuators are currently available; some are used in the medical field for osteo treatment and some are used in the mechanical processing field for drilling and cutting a bone. Any of those remote controlled actuators controls by remote control a machine tool fitted to a distal end of an elongated pipe of a linear or curved configuration. However, since the conventional remote controlled actuator is designed solely to control only the rotation of the machine tool by remote control, difficulties have been encountered in processing of a complicated shape and processing at a site difficult to view with eyes from the outside in the medical field. Also, in the drilling process, the capability of processing not only the linear line, but also the curved configuration is often required. In addition, in the cutting process, the capability is required to perform the process at a site deep in grooves. In the following description, conventional art and problems inherent in the remote controlled actuator will be discussed with reference to the medical field.

In the orthopedic field, the artificial joint replacement is well known, in which a joint, of which bone has been abraded by due to bone deterioration, is replaced with an artificial joint. The joint replacement surgery requires a living bone of a patient to be processed to enable an artificial joint to be implanted. In order to enhance the strength of postoperative adhesion between the living bone and the artificial joint, such processing is required to be performed precisely and accurately in conformity to the shape of the artificial joint.

By way of example, during the hip join replacement surgery, a thigh bone is opened to secure access of an artificial joint into the femoral marrow cavity. In order to secure a strength of contact between the artificial joint and the bone, surfaces of contact of the artificial joint and the bore must be large and so the opening for insertion of the artificial joint is processed to represent an elongated shape extending deep into the bone. As a medical actuator used in cutting the bone in a manner described above, the actuator is known, in which a tool is rotatably provided in a distal end of an elongated pipe and, on the other hand, a drive source such as, for example, a motor is mounted on a proximal end of the pipe so that the tool can be driven through a rotary shaft disposed inside the elongated pipe. (See, for example, the Patent Document 1 listed below.) Since in this type of medical actuator a rotatable element that is exposed bare to the outside is only the tool at the distal end of the elongated pipe, the tool can be inserted deep into the bone.

The surgical operation for artificial joint replacement generally accompanies skin incision and muscular scission. In other words, the human body must be invaded. In order to minimize the postoperative trace, it is quite often desirable that the elongated pipe referred to above is not necessarily straight, but is moderately curved. To meet with this desire, the following technique has hitherto been suggested. For example, the Patent Document 2 listed below discloses the elongated pipe having its intermediate portion curved double to displace an axial position of the distal end of the pipe relative to the longitudinal axis of the proximal end of the same pipe. To make the axial position of the distal end of the pipe relative to the longitudinal axis of the proximal end of the same pipe is also known from other publications. Also, the Patent Document 3 listed below discloses the elongated pipe rotated 180°.

If in a condition, in which the artificial joint is inserted into an artificial joint insertion hole formed in the living bone, a large gap exist between the living bone and the artificial joint, a large length of time is required to accomplish the postoperative adhesion between the living bone and the artificial joint and, therefore, it is considered desirable that the gap should be as small as possible. Also, it is important that respective surfaces of contact between the living bone and the artificial joint be smooth, and accordingly, a high precision is required in processing the artificial joint insertion hole. Whatever the pipe take any shape, the working range of the tool is limited by the shape of the pipe and, therefore, it is difficult to widen the working range of the tool to process the artificial joint insertion hole so that the living bone and the artificial joint may can have smooth contact surfaces and, yet, the gap between the living bone and the artificial joint may be small while skin incision and muscular scission are minimized at the same time.

In general, it is quite often that the patient's bone, where an artificial joint is to be implanted, exhibits a strength lowered as a result of aging and, in a certain case, the bone itself is deformed. Accordingly, the processing of the artificial joint insertion hole is more difficult to achieve than generally considered.

In view of the foregoing, the applicant or assignee of the present invention has attempted to provide a remote control actuator of a type designed to enable the processing of the artificial joint insertion hole to be relatively easily and accurately accomplished. For this purpose the remote controlled actuator of the type referred to above includes an elongated spindle guide section of a pipe-like contour, provided in an actuator main body, and a distal end member provided at a distal end portion of the spindle guide section for rotatably supporting a processing tool in a fashion capable of being altered in attitude so that the distal end member can be altered in attitude by remote control. This is because if the attitude of the tool can be changed, the tool can be maintained at a proper attitude regardless of the shape of the pipe. It is eventually pointed out that although the medical actuator of a type having no elongated pipe such as, for example, the spindle guide section, in which a portion provided with a processing tool is changeable in attitude relative to the grip that is held by hand, is currently available in the medical field (See, for example, the Patent Document 4 listed below.), nothing has yet been suggested which has a capability of changing the attitude of the tool by remote control.

Where the artificial joint insertion hole is to be processed in the bone with the use of the remote controlled actuator, it is quite often that the tool cannot be directly viewed with eyes and, therefore, a navigation system is needed in order to grasp the position of the tool. For this type of the navigation system, the following techniques have hitherto been well known in the art.

The Patent Document 5 listed below discloses the navigation system, in which a marker is applied to the bone so that the position of the bone can be measured by the detection of the marker with the use of an optical sensor. Using this technique, when a marker is applied not only to the bone, but also to the body of a remote controlled actuator, which is a stationary portion of such actuator in such case, respective positions of the bone and the actuator main body can be measured.

The Patent Document 6 also listed below discloses the navigation system, in which a marker, which is formed in a specific pattern, not in a dot, is applied to the actuator main body so that both of the position of the marker and the attitude of the actuator main body to which the marker has been applied can be detected by the detection of the pattern of that marker with the use of a marker detecting machine. Once the attitude of the actuator main body is determined, the position of the tool can be estimated from the relative positional relation between the distal end member and the tool and the site of the actuator main body where the marker has been applied. It is, however, to be noted that the relative positional relation between the distal end member and the tool and the site of the actuator main body where the marker has been applied is measured beforehand and is recorded and stored as a positional relational information.

It occurs quite often that the tool is replaced with a different type thereof depending on an object to be processed and/or upon, for example, wear, and, therefore, the positional relational information to be used in estimation of the position of the tool is needed to be updated each time the replacement takes place. As such the Patent Document 6 discloses the use of a switch operatively linked with removal of the tool to provide a piece of information with which the operator of the remote controlled actuator can be informed of the necessity of updating of the positional relational information.

The Patent Document 7 listed below discloses the navigation system, in which a second marker different from a first marker fitted to the actuator main body is prepared for use and in which the tool is brought into contact with the second marker and, using a positional relation between the actuator main body and the second marker then measured, the position of the tool is estimated on the basis of the positional relation between the actuator main body and the first marker measured during the procedure.

### [Prior Art Literature]

[Patent Document 1] JP Laid-open Patent Publication No. 2007-301149
[Patent Document 2] United State Patent No. 4,466,429
[Patent Document 3] United State Patent No. 4,265,231
[Patent Document 4] JP Laid-open Patent Publication No. 2001-17446
[Patent Document 5] United State Patent No. 5,249,581
[Patent Document 6] United State Patent No. 6,434,507
[Patent Document 7] United State Patent No. 7,166,114

The above mentioned navigation system is premised on the tool having been fixed relative to the actuator main body and, therefore, there has been found a problem that such navigation system is inapplicable to the remote controlled actuator of the type having the distal end member that can rotatably support the tool relative to the actuator main body in a fashion capable of altering in attitude thereof.

### SUMMARY OF THE INVENTION

The present invention is therefore intended to provide a navigation system applicable to a remote controlled actuator of a type, in which the attitude of a distal end member provided at a distal end portion of an elongated spindle guide section of a pipe-like configuration in appearance for supporting a tool can be altered by a remote control, which system is designed to enable the position of the tool to be estimated.

To describe a navigation system for a remote controlled actuator according to the present invention with the aid of reference numerals, used in the accompanying drawings, for facilitating a better understanding thereof, the remote controlled actuator includes an actuator main body 10, in which a base end of a spindle guide section 3 of an elongated configuration is connected with a drive unit housing 4a; a distal end member 2 fitted to a distal end portion of the spindle guide section 3 for pivotal movement about a pivot center O to enable it to be altered in attitude; a tool 1 rotatably supported by the distal end member 2; an attitude altering drive source 42 and a tool rotation drive source 41 both provided within the drive unit housing 4a for altering the attitude of the distal end member 2 and rotating the tool 1, respectively; and an operator unit 50 provided in the drive unit housing 4a for controlling respective operations of the drive sources 42 and 41 for maneuvering the attitude of the distal end member 2 and the rotation of the tool 1, to thereby estimate the position of a processing member 1a of the tool 1. The navigation system includes a marker detecting unit 8 for detecting respective positions and attitudes of a main body marker 7A, fitted to the drive unit housing 4a of the actuator main body 10, a tool attitude detector 45 for detecting the attitude of the distal end member 2 relative to the actuator main body 10, an actuator shape storage section 53, and a tool processing member position estimator 55. The actuator shape storage section 53 referred to above stores information on the relative position of the pivot center O1 relative to the main body marker 7A and information on the shape of the tool 1 with reference to the pivot center O1. The tool processing member position estimator 55 estimate the position of a processing member 1a of the tool 1 from information on the position and attitude of the main body marker 7A, detected by the marker detecting unit 8, the information on the relative position of the pivot center O1 and the information on the shape of the tool 1, both stored in the actuator shape storage section 53, and information on the attitude of the distal end member 2 detected by the tool attitude detector 45. It is to be noted that the information on the shape of the tool 1 stored in the actuator shape storage section 53 is satisfactory if the distance from the pivot center O1 to the processing member 1a of the tool 1 can be recognized with it, or alternatively it may be information only on the distance referred to above.

According to the above described construction, the marker detecting unit 8 detects the position and attitude of the marker 7A fitted to the drive unit housing 4a of the actuator main body 10. Accordingly, the position and attitude of the reference portion of the actuator main body 10 is detected. The tool attitude detector 45 detects the attitude of the distal end member 2 relative to the actuator main body 10. The tool processing member position estimator 55 estimates the position of the processing member 1a of the tool 1 from the information on the position and attitude of the marker 7A detected by the marker detecting unit 8, the information on the relative position of the center of pivot O1 of the distal end member 2 and the information on the shape of the tool 1, both stored in the actuator shape storage section 53, and the information on the attitude of the distal end member 2 detected by the tool attitude detector 45.

In other words, the tool processing member position estimator 55 can estimate the absolute position of the pivot center O1 from the information on the position and attitude of the marker 7A detected by the marker detecting unit 8, that is, the position and attitude of the reference portion of the actuator main body 10 and the information on the relative position of the center of pivot O1 of the distal end member 2 relative to the marker 7A stored in the actuator shape storage section 53. Also, the relative position of the processing member 1a of the tool 1 relative to the pivot center O1 can be estimated from the information on the attitude of the distal end member 2 relative to the actuator main body 10 detected by the tool attitude detector 45 and the information on the shape of the tool 1 stored in the actuator shape storage section 53.

From the absolute position of the center of pivot O1 of the distal end member 2 so estimated as described above and the relative position of the processing member 1a of the tool 1 with the position of the pivot center O1 taken as a reference, the absolute position of the processing member 1a of the tool 1 can be estimated. For this reason, relative to the remote controlled actuator 5 capable of performing a remote control of the alteration of the attitude of the distal end member 2 for the support of the tool 1, which is provided at the distal end of the spindle guide section 3, the position of the processing member 1a of the tool 1 can be estimated.

In the present invention, in order that a plurality of remote controlled actuators 5 utilizing respective different types of spindle guide sections 3 to be useable, it is recommended that the actuator shape storage section 53 may store the information on the relative position of the pivot center O1 relative to the marker for each type of the spindle guide sections 3, and that the use is made of a spindle guide section type selector 53a for selecting the information on the relative position of the pivot center O1 that is to be used in estimation performed by the tool processing member position estimator 55.

In the case where a certain remote controlled actuator 5 is to be used, out from the information on the relative position of the pivot center O1 relative to the marker 7A that is stored in the actuator shape storage section 53, the information corresponding to the type of the spindle guide section 3 of the remote controlled actuator 5 is selected by the spindle guide section type selector 53a. By so doing, relative to the plural types of the remote controlled actuators 5 utilizing the different types of the spindle guide sections 3, the navigation system can become useable.

In the present invention, in order that a plurality of remote controlled actuators 5, utilizing respective different types of tools 1, and the remote controlled actuator 5 of a type, in which different types of the tools 1 can be replaced one at a time, to be useable, it is recommended that the actuator shape storage section 53 may store the information on the shape of the tool 1 for each type of the tool 1 with reference to the pivot center O1, and that the use may be made of a tool type selector 53b for selecting the information on the shape of the tool 1 that is to be used in estimation performed by the tool processing member position estimator 55.

In the case where a certain remote controlled actuator 5 is to be used, out from the information on the shape of the tool 1 with the pivot center O1 taken as a reference, which is stored in the actuator shape storage section 53, the information corresponding to the type of the tool 1 of the remote controlled actuator 5 is selected by the tool type selector 53b. By so doing, relative to the plural types of the remote controlled actuators 5 utilizing the different types of the tools 1, the navigation system can become useable.

In the present invention, the marker 7A is of a type capable of projecting or reflecting light and the marker detecting unit 8 is of an optical type capable of receiving the light from the marker. The marker detecting unit 8 of the optical type has a simplified structure.

In the present invention, the tool attitude detector 45 may be provided in the attitude altering drive source 42 or a drive system for transmitting an operation from the attitude altering drive source 42 to the distal end member 2 and is operable to output an electric signal corresponding to the attitude of the distal end member 2.

If the operation of the distal end member 2 is outputted in the form of the electrical signal, the transmission of information between the remote controlled actuator and a control system portion of the navigation system can be facilitated particularly where the remote controlled actuator and the control system portion are separated a distance from each other.

In the present invention, a display unit 52 may be provided for displaying images such that the tool processing member position estimator 55 is provided with an actuator display information generator 55a for calculating an actuator display information, which is information for displaying the position and attitude of the actuator main body 10 and the attitude of the distal end member 2, from various input information that is used in the estimation of the position of the tool 1, and then displaying such actuator display information on a screen of the display unit 52.

If the display unit 52 and the actuator display information generator 55a are employed, the position and attitude of the actuator main body 10 and the actuator display information, which is information for displaying the attitude of the distal end member 2, can be displayed on the screen of the display unit 52 and, therefore, the operator can readily recognize such information during the manipulation of the remote controlled actuator 5.

Where the display unit 52 and the actuator display information generator 55a are employed, the actuator display information generated by the actuator display information generator 55a may be information necessary to display the position and attitude of the actuator main body 10 and the attitude of the distal end member 2 on the screen of the display unit in the form of series of dots 60.

Display in the form of the dots 60 makes it possible for the operator to recognize visually and also facilitate the calculation taking place in the actuator display information generator 55a.

Also, the actuator display information generator 55a may be of a type generating, as the actuator display information, a graphic symbol 61 and then displaying the graphic symbol 61 on the screen of the display unit 52, which symbol 61 is representative of an external shape of the tool 1, the distal end member 2 and the actuator main body 10, which reflect the position and attitude of them, by means of a computer graphics.

If the graphic symbol 61 representative of the outer shape is displayed, the visual recognition can be further facilitated.

Furthermore, the actuator display information generated by the actuator display information generator 55a may be information in the form of a numeral on the screen of the display unit 52.

In any case, by displaying on the screen of the display unit 52, the position and attitude of the actuator main body 10 and the actuator display information which is the information for displaying the position of the distal end member 2, the operator can be informed of such information.

The remote controlled actuator is preferably constructed as follows. That is to say, the distal end member rotatably supports the spindle for holding the tool; the spindle guide section includes a rotatable shaft for transmitting rotation of the tool rotation drive source within the drive unit housing to the spindle and an attitude altering member for altering the attitude of the distal end member by selectively advancing or retracting by means of the attitude altering drive source within the drive unit housing in a condition with the tip end held in contact with the distal end member.

If the remote controlled actuator is of the structure described above, the rotation of the tool rotation drive source within the drive unit housing is transmitted to the spindle through the rotary shaft accommodated within the spindle guide section and the attitude altering member arranged within the spindle guide section is selectively advanced or retracted by the attitude altering drive source within the drive unit housing, to thereby alter the attitude of the distal end member. Accordingly, the rotation of the tool and the alteration of the attitude of the distal end member can be accomplished by remote control.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims. In the accompanying drawings, like reference numerals are used to denote like parts throughout the several views, and:
Fig. 1 is a diagram showing a schematic structure of a navigation system for a remote controlled actuator according to a first preferred embodiment of the present invention;
Fig. 2A is a sectional view showing a distal end member and a spindle guide section of the remote controlled actuator according to the first embodiment of the present invention;
Fig. 2B is a cross sectional view taken along the line IIB-IIB in Fig. 2A;
Fig. 2C is a diagram showing a connecting structure between the distal end member and a rotary shaft;
Fig. 3A is a side view showing a tool rotation drive mechanism and an attitude altering drive mechanism both employed in the remote controlled actuator;
Fig. 3B is a view taken in the direction of an arrow IIIB-IIIB in Fig. 3A;
Fig. 4 is a block diagram showing a control system of the navigation system;
Fig. 5A is a diagram showing a tool, a distal end member and a spindle guide section, all employed in the remote controlled actuator;
Fig. 5B is a diagram showing the tool, the distal end member and the spindle guide section of a different shape;
Fig. 6 is a diagram showing a structure of a portion of an actuator shape storage section employed in the navigation system;
Fig. 7A is a diagram showing the tool, the distal end member and the spindle guide section all employed in the remote controlled actuator;
Fig. 7B is a diagram showing a structure of the tool of a different type, the distal end member and the spindle guide section;
Fig. 8 is a diagram showing a structure of a portion of the actuator shape storage section employed in the navigation system;
Fig. 9 is a diagram showing an example of a display on a screen of a display unit employed in the navigation system;
Fig. 10 is a diagram showing a different example of a display on a screen of a display unit employed in the navigation system;
Fig. 11A is a sectional view showing the distal end member and the spindle guide section employed in the remote controlled actuator according to a second preferred embodiment of the present invention;
Fig. 11B is a cross sectional view taken along the line XIB-XIB in Fig. 11A;
Fig. 12A is a sectional view showing the distal end member and the spindle guide section employed in the remote controlled actuator according to a third preferred embodiment of the present invention;
Fig. 12B is a cross sectional view taken along the line XIIB-XIIB in Fig. 12A;
Fig. 13 is a front elevational view showing the tool rotation drive mechanism and the attitude altering drive mechanism both employed in the remote controlled actuator according to the third embodiment of the present invention;
Fig. 14 is a schematic structural diagram showing a condition in which the navigation system for the remote controlled actuator is used in processing according to a first mode of application of the present invention;
Fig. 15A is a schematic structural diagram showing a condition of the navigation system being tested according to the first mode of application of the present invention;
Fig. 15B is a diagram showing a portion of Fig. 15A on an enlarged scale;
Fig. 16 is a block diagram showing the control system of the navigation system;
Fig. 17 is a diagram showing a structure of a tool marker relative position and attitude storage section employed in the navigation system;
Fig. 18 is a diagram showing a structure of a tool and tool marker relative position storage section;
Fig. 19A is an explanatory diagram showing a positional relation between a reference point of the tool and a reference point of the tool marker when a tool is mounted on the distal end member;
Fig. 19B is an explanatory diagram showing a positional relation between a reference point of the tool and a reference point of the tool marker when a different tool is mounted on the distal end member;
Fig. 20 is a diagram showing a structure of a portion of the navigation system unit of a different navigation system;
Fig. 21A is a schematic structural diagram showing a condition of the navigation system being calibrated; and
Fig. 21B is a fragmentary enlarged view of Fig. 21A.

### DESCRIPTION OF PREFERRED EMBODIMENTS

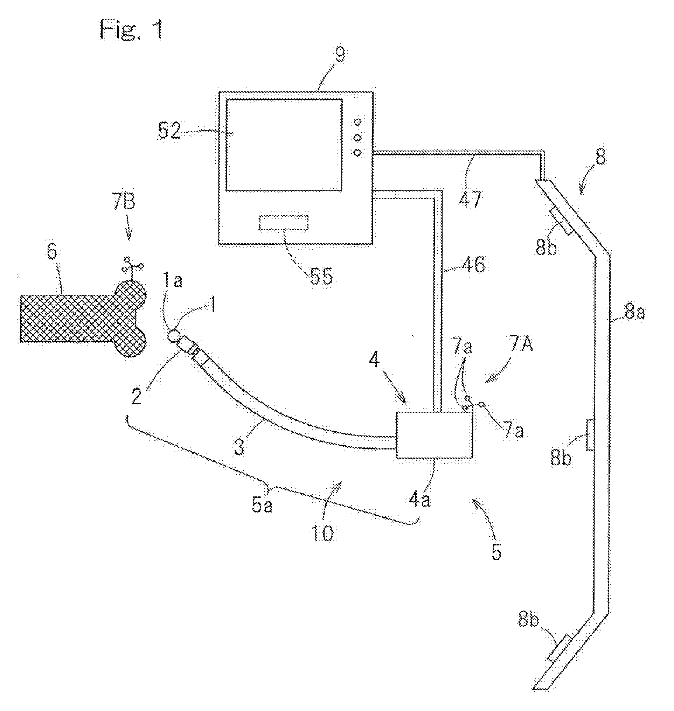

Fig. 1 illustrates a schematic structure of a navigation system for a remote controlled actuator in accordance with a first preferred embodiment of the present invention. The illustrated navigation system is applied to the remote controlled actuator 5 of a type capable of navigating the rotation and the attitude of a tool 1 by remote control and includes a marker detecting unit 8 for detecting the position of and the attitude of markers 7A and 7B fitted respectively to the remote controlled actuator 5 and an object to be processed 6, and a navigation computer 9 for concurrently performing a control of the navigation system and a control of operation of the remote controlled actuator 5.

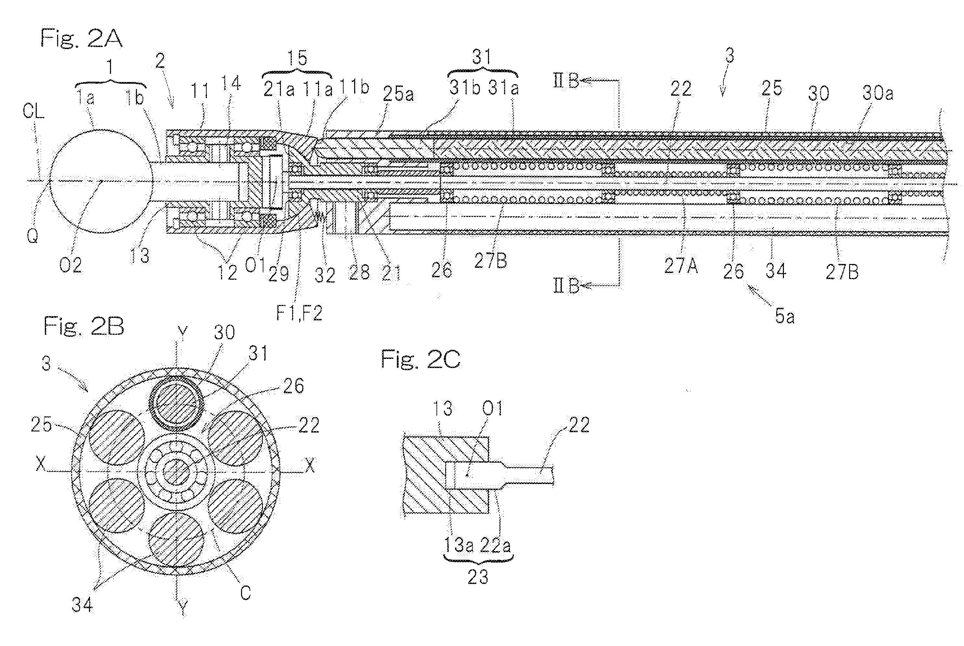

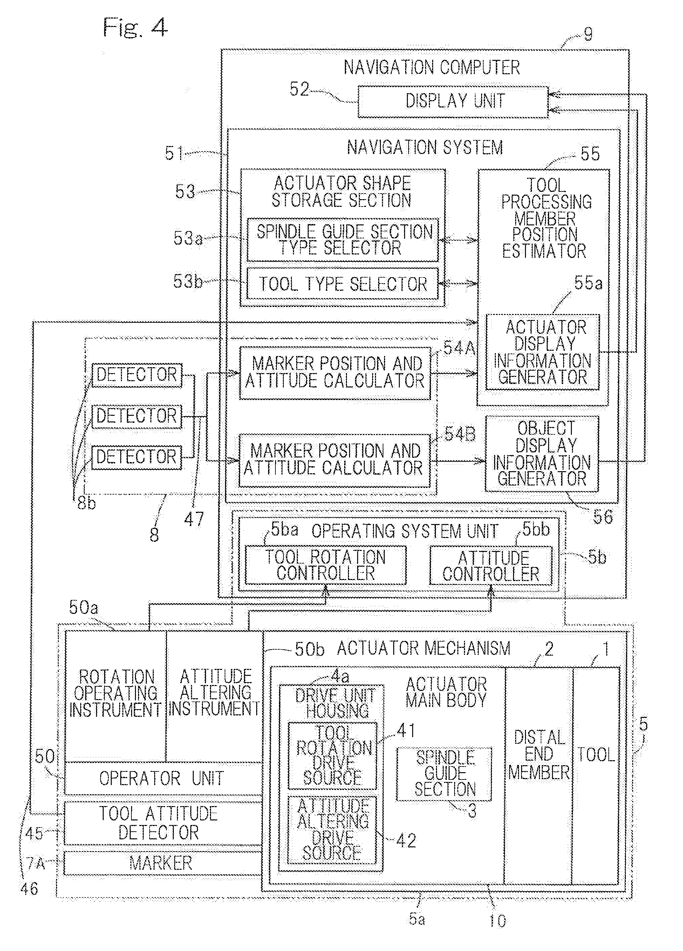

The remote controlled actuator 5 includes an actuator mechanism 5a, best shown in Fig. 1 and Fig. 2A, and an operating system unit 5b, best shown in Fig. 4. Hereinafter, the details of the actuator mechanism 5a will be described with particular reference to Fig. 1 to Fig. 3B. It is, however, to be noted that while Fig. 2A illustrates a spindle guide section 3 of a linear configuration, but the spindle guide section 3 can have a basically identical structure regardless of whether it has a linear shape as shown in Fig. 2A, whether it has a curved shape as shown in Fig. 1A or whether it has any other shape.

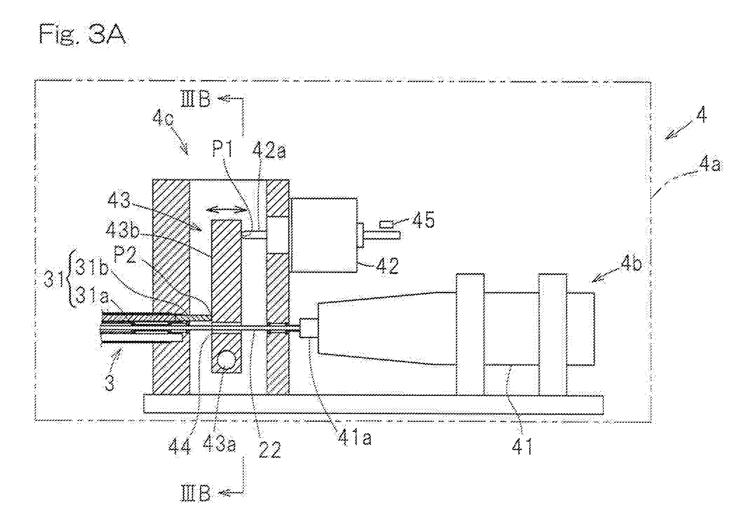

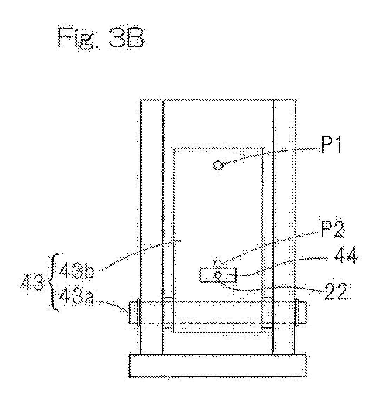

Referring now to Fig. 1, the actuator mechanism 5a includes a distal end member 2 for holding a rotary tool 1, the elongated spindle guide section 3 of a pipe-like appearance having its distal end to which the distal end member 2 is fitted for alteration in attitude, and a drive unit housing 4a to which a proximal end of the spindle guide section 3, opposite to the above mentioned distal end, is connected. The drive unit housing 4a cooperates with a built-in tool rotation drive mechanism 4b, best shown in Fig. 3A, and a similarly built-in attitude altering drive mechanism 4c to form a drive unit 4. Also, the spindle guide section 3 and the drive unit 4 altogether constitute an actuator main body 10. The drive unit housing 4a is provided with an operator unit 50 that is made up of a rotation operating instrument 50a, best shown in Fig. 4, for rotating the tool 1 by controlling the operation of the tool rotation drive mechanism 4b and an attitude altering instrument 50b, also best shown in Fig. 4, for effecting alteration of the attitude of the distal end member 2 by controlling the operation of the attitude altering drive mechanism 4c.

As shown in Fig. 2A, the tool 1 is made up of the processing member 1a and a shank 1b. In the embodiment now under discussion, the processing member 1a is of a spherical shape. The distal end member 2 includes a generally or substantially cylindrical housing 11 and a spindle 13 rotatably accommodated within such cylindrical housing 11 through a pair of bearings 12. The spindle 13 is of a tubular shape having a distal side opening and having a hollow defined therein, and a tool 1 is drivingly coupled with the spindle 13. Specifically, a shank 1b of the tool 1 is inserted into the hollow of the spindle 13 in a removable fashion and is then coupled with such spindle 13 by means of a stop pin 14 for rotation together with the spindle 13. The distal end member 2 of the structure described above is coupled with a distal end of the spindle guide section 3 through a distal end member connecting unit 15. The distal end member connecting unit 15 is means for supporting the distal end member 2 for displacement in attitude and is comprised of a spherical bearing. More specifically, the distal end member connecting unit 15 includes a guided member 11a in the form of an inner diameter reduced portion at a base end of the housing 11, and a guide member 21a in the form of a collar integral with a constraint member 21 fixed to the tip of the spindle guide section 3. The guided member 11a and the guide member 21a have respective guide faces F1 and F2 that are held in sliding contact with each other, and those guide faces F1 and F2 have respective centers of curvature lying at a point O1 on the center line or longitudinal axis CL of the spindle 13, having their diameters being reduced towards the base end of the spindle 13. Accordingly, not only can the distal end member 2 be immovably constrained relative to the spindle guide section 3, but it can also be supported for displacement in attitude so that the attitude of the distal end member 2 can be altered. It is to be noted that since in this example, the distal end member 2 can have its attitude altered about a lateral X-axis passing through the center of curvature O1, the guide faces F1 and F2 may be a cylindrical surface having a longitudinal axis represented by the X-axis passing through the center of curvature O1.

The spindle guide section 3 includes a rotary shaft 22 for transmitting a rotational force exerted by a tool rotation drive source 41 accommodated within the drive unit housing 4a (Fig. 3A). In the illustrated example, the rotary shaft 22 is employed in the form of a wire capable of undergoing deformation to a certain extent. Material for the wire includes, for example, metal, resin or glass fiber. The wire may be either a single wire or a stranded wire. As best shown in Fig. 2C, the spindle 13 and the rotary shaft 22 are connected together by means of a universal joint 23 for transmitting rotation from the rotary shaft 22 to the spindle 13. The universal joint 23 is made up of a groove 13a, defined in a closed base end of the spindle 13, a projection 22a defined in a distal end of the rotary shaft 22 and engageable in the groove 13a. The center of joint between the groove 13a and the projection 22a is located at the same position as the centers of curvature O1 of the guide faces F1 and F2.

The spindle guide section 3 includes an outer shell pipe 25 forming an outer shell of the spindle guide section 3 and the rotary shaft 22 referred to above is positioned at the center of this outer shell pipe 25. The rotary shaft 22 so positioned is rotatably supported by a plurality of rolling bearings 26 positioned spaced a distant apart from each other in a direction axially of the spindle guide section 3. Spring elements 27A and 27B for generating a preload on the corresponding rolling bearing 26 are disposed between the neighboring rolling bearings 26. Each of those spring elements 27A and 27B is employed in the form of, for example, a compression spring. There are the spring element 27A for inner ring for generating the preload on the inner ring of the rolling bearing 26 and the spring element 27B for outer ring for generating the preload on the outer ring of the rolling bearing 26, and the both are arranged alternately relative to each other. The constraint member 21 referred to previously is fixed to a pipe end portion 25a of the outer shell pipe 25 by means of a fixing pin 28 and has its distal end inner peripheral portion supporting the distal end of the rotary shaft 22 through a rolling bearing 29. It is, however, to be noted that the pipe end portion 25a may be a member separate from the outer shell pipe 25 and may then be connected with the outer shell pipe 25 by means of, for example, welding.

A single guide pipe 30 open at opposite ends thereof is provided between an inner diametric surface of the outer shell pipe 25 and the rotary shaft 22, and an attitude altering member 31, made up of a wire 31a and pillar shaped pins 31b at opposite ends, is axially movably inserted within a guide hole 30a, which is an inner diametric hole of the guide pipe 30. One of the pillar shaped pins 31b, which is on the side of the distal end member 2, has its tip representing a spherical shape and is held in contact with a base end face of the distal end member housing 11. The base end face 11b of the housing 11, which defines a surface of contact between the distal end member 2 and the attitude altering member 31, for the distal end member 2 is so shaped as to represent an inclined face such that an outer peripheral edge thereof is closer to the spindle guide section 3 than a center portion thereof. Similarly, as best shown in Fig. 3A, the other of the pillar shaped pins 31b, that is, the pillar shaped pin 31b on the side of the drive unit housing 4a has its tip representing a spherical shape and held in contact with a side face of a lever 43 as will be described in detail later.

It is to be noted that the use of the pillar shaped pins 31b may be dispensed with, leaving only the signal wire 31a to constitute the attitude altering member 31.

At a position spaced 180° in phase from a peripheral position where the attitude altering member 31 referred to above is positioned, a restoring elastic member 32, which is in the form of, for example, a compression spring, is provided between the base end face of the housing 11 for the distal end member 2 and a tip end face of the outer shell pipe 25 of the spindle guide section 3. This restoring elastic member 32 has a function of biasing the distal end member 2 towards the side of a predetermined attitude.

Also, as best shown in Fig. 2B, between the inner diametric surface of the outer shell pipe 25 and the rotary shaft 2, a plurality of reinforcement shafts 34 are arranged separate from the guide pipe 30 and on the pitch circle C of the same diameter as the guide pipe 30. Those reinforcement shafts 34 are used to secure the rigidity of the spindle guide section 3. The guide pipe 30 and the reinforcement shafts 34 are arranged equidistantly relative to each other around the rotary shaft 22. The guide pipe 30 and the reinforcement shafts 34 are held in contact with the inner diametric surface of the outer shell pipe 25 and respective outer peripheral surfaces of the rolling bearings 26. In this manner, the outer diametric surfaces of those rolling bearings 26 are supported.

The tool rotation drive mechanism 4b includes the tool rotation drive source 41 referred to previously. The tool rotation drive source is in the form of, for example, an electrically driven motor having its output shaft 41a coupled with a base or proximal end of the rotary shaft 22.

The attitude altering drive mechanism 41 includes an attitude altering drive source 42. This attitude altering drive source 42 is in the form of, for example, an electrically operated linear actuator and had an output rod 42a capable of moving leftwards or rightwards, as viewed in Fig. 3A, the movement of such output rod 42a being transmitted to the attitude altering member 31 through a lever mechanism 43, which is a force transmitting mechanism. It is to be noted the attitude altering drive source 42 may be a rotary motor. The amount of actuation of the attitude altering drive source 42 is detected by a tool attitude detector 45. A detection signal outputted from this tool attitude detector 45 is supplied to a tool processing member position estimator 55 (best shown in Fig. 4) of the navigation computer 9 through an actuator electric cable 46 (best shown in Fig. 1 and Fig. 4).

The lever mechanism 43 includes a pivot lever 43b pivotable about a support pin 43a and is so designed and so configured as to allow a force of the output rod 42a to work on a working point P1 of the lever 43b, which is spaced a long distance from the support pin 43a, and as to apply a force to the attitude altering member 31 at a force point P2, which is spaced a short distance from the support axis 43a, wherefore an output of the attitude altering drive source 42 can be increased and then transmitted to the attitude altering member 31. Since the use of the lever mechanism 43 is effective to enable a large force to be applied to the attitude altering member 31 even in the linear actuator of a low output capability, the linear actuator can be downsized. The rotary shaft 22 extends through an opening 44 defined in the pivot lever 43b. It is to be noted that instead of the use of the attitude altering drive source 42 or the like, the attitude of the distal end member 2 may be manually operated from a remote site (by remote control).

The marker detecting unit 8 shown in Fig. 1 includes individual detectors 8b supported by a detector support body 8a and marker position and attitude calculators 54A and 54B within the navigation computer 9 as best shown in Fig. 4. The main body marker 7A is fitted to the drive unit housing 4a, which forms a part of the actuator main body 10. The to-be-processed object marker 7B is fitted to the object to be processed 6 such as, for example, a bone. In correspondence with the individual detectors 8b of the marker detecting unit 8, each of the markers 7A and 7B is provided with three light reflectors 7a. Those three light reflectors 7a are disposed at different positions, respectively.

Each of the marker detectors 8b is of an optical type and is so designed and so configured as to project a detection beams towards the light reflectors 7a of each of the markers 7A and 7B and then receive rays of light reflected from those light reflectors 7a. Respective detection signals of those marker detectors 8b are supplied to respective marker position and attitude calculators 54A and 54B (best shown in Fig. 4) in the navigation computer 9 through a wiring system (not shown), built in the detector support body 8a, and a marker detector electric cables 47. It is, however, to be noted that the use of three light projectors (not shown) may be provided respectively in the markers 7A and 7B so that detection beams projected from those light projectors can be received by the individual detectors 8b. The use of the marker detectors 8b of an optical type as discussed above is effective to allow the marker detecting unit 8 to be assembled portable. It is, however, also to be noted that each of the marker detectors 8b may not necessarily be of an optical type and may be of, for example, a magnetic type.

As shown in Fig. 4, the navigation computer 9 includes a navigation system unit 51 and a display unit 52. The navigation system unit 51 is comprised of hardware of the navigation and maneuvering computer 9 and a software program executed thereby, or comprised of a further addition of an electronic circuit.

The operating system unit 5b is made up of a tool rotation controller 5ba and an attitude controller 5bb. The operating system unit 5b is comprised of hardware and a software program executed thereby, or comprised of a further addition of an electronic circuit. The tool rotation controller 5ba provides an output to a motor driver (not shown) in response to an input from a rotation operating instrument 50a so as to drive the tool rotation drive source 41. The attitude controller 52b provides an output to the motor driver (not shown) in response to an input from an attitude altering instrument 50b to thereby drive the attitude altering drive source 42.

The navigation system unit 51 includes an actuator shape storage section 53, marker position and attitude calculators 54A and 54B, and a tool processing member position estimator 55. The actuator shape storage section 53 referred to above in turn includes a spindle guide section type selector 53a and a tool type selector 53b. The tool processing member position estimator 55 includes an actuator display information generator 55a. Also, separate from them, the navigation system unit 51 includes a to-be-processed object display information generator 56.

The actuator shape storage section 53 is operable to store therein information on the relative position of the pivot center O1 of the distal end member 2 relative to the main body marker 7A fitted to the drive unit housing 4a and information on the shape of the tool 1 with reference to the pivot center O1. For the information on the shape of the tool 1, information can be employed, which pertains to the relative position of the center 02 (shown in Fig. 2A) of the processing member 1a relative to the pivot center O1 when the attitude of the distal member 2 held at, for example, a neutral position. This information on the relative position may be merely the distance between the pivot center O1 of the distal end member 2 and the center 02 of the processing member 1a.

The actuator shape storage section 53 will be described in further details hereinafter.

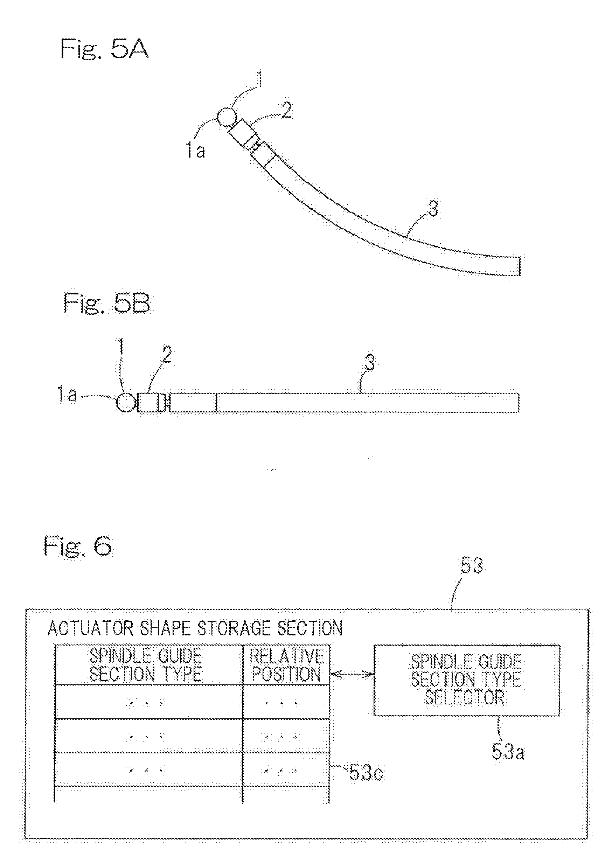

The relative position of the pivot center O1 of the distal end member 2 relative to the main body marker 7A depends on the shape of the spindle guide section 3. By way of example, depending on whether the spindle guide section 3 used is of a curved configuration as best shown in Fig. 5A or whether the spindle guide section 3 is of a linear configuration as best shown in Fig. 5B, the relative position referred to above differs. Even when the spindle guide section 3 is deformed, for example, artificially, the relative position referred to above differs before and after the deformation. The actuator shape storage section 53 accommodates therein a table 53c recording and storing relations between the types of the spindle guide sections 3 and the relative positions, and from those plural relations stored and preserved in this table 53c, the spindle guide section type selector 53a selects a proper one of those relations in dependence on information inputted from outside. The relative positions of the spindle guide section 3 for each of those types is determined in reference to design data measured beforehand.

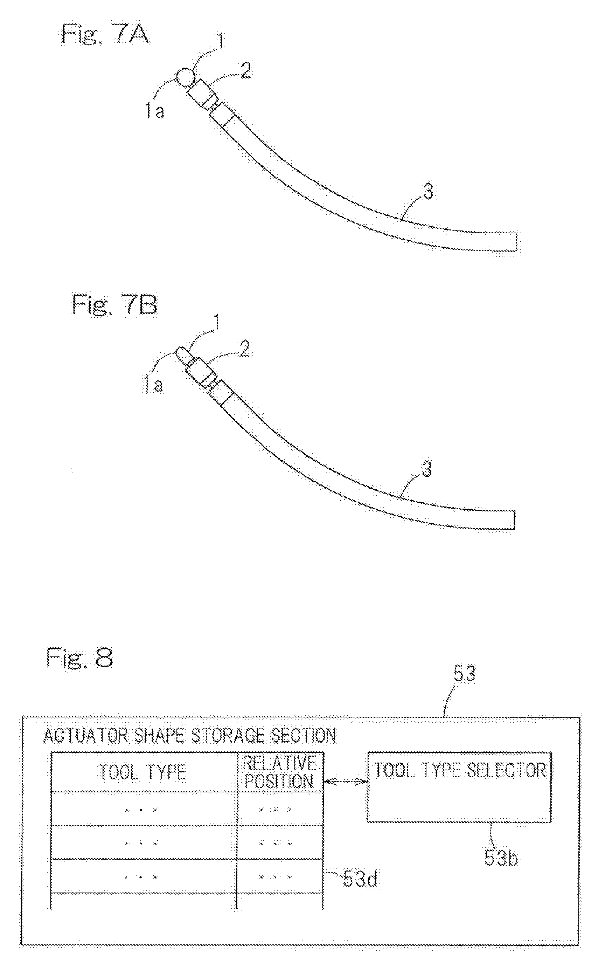

Also, the shape of the tool 1 with reference to the pivot center O1 differs depending on the type of the tool 1 used. By way of example, depending on whether the tool 1 is employed in the form of the type having the spherical processing member 1a as best shown in Fig. 7A or whether the tool 1 is employed in the form of the type having a pillar shaped configuration as best shown in Fig. 7B, the relative position referred to above differs. The actuator shape storage section 53 accommodates therein a table 53d storing and preserving relations between the types of the tools 1 and the relative positions, and from those plural relations stored and preserved in this table 53d, the tool type selector 53b selects a proper one of the relations in dependence on information inputted from outside. The relative position of the tool 1 for each of those types is determined in reference to design data or measurements beforehand. Information concerning the shape of the tool 1 may be employed, for example, in the form of information on the relative position of a processing end Q of the processing member 1a relative to the pivot center O1 when the attitude of the distal end member 2 is in the neutral position. The processing end Q referred to above is a tip end of the tool 1 lying on a rotational center line (the center line of the spindle 13) CL and is a site that is mainly held in contact with the to-be-processed object 6.

The marker position and attitude calculator 54A shown in Fig. 4 is operable to calculate the position and the attitude of the main body marker 7A, fitted to the drive unit housing 4a shown in Fig. 1, from detection signals of the individual detectors 8b of the marker detecting unit 8. With the light reflectors 7a of the marker 7A as well as the individual detectors 8b of the marker detecting unit 8 being employed in three or more in number, the three dimensional position and attitude of the marker 7A can be determined. The position and the attitude of the main body marker 7A are analogous to the position and the attitude of the actuator main body 10. In other words, by the marker position and attitude calculator 54A shown in Fig. 4, the position and attitude of a reference portion of the actuator main body 10 is detected. The term "reference portion" referred to above and hereinafter is intended to mean a portion that provides a basis for calculation performed by the tool processing member position estimator 55 as will be described later.

Similarly, the marker position and attitude calculator 54B is operable to calculate the position and the attitude of the to-be-processed object marker 7B, fitted to the to-be-processed object 6 best shown in Fig. 1, from the detection signals of the individual detectors 8b of the marker detecting unit 8. The position and attitude of the to-be-processed object marker 7B is analogous to the position and the attitude of the to-be-processed object 6.

The tool processing member position estimator 55 shown in Fig. 4 is operable to estimate the position of the processing member 1a of the tool 1 from the information on the position and attitude of the main body marker 7A which has been determined by the marker position and attitude calculator 54A, the information on the relative position of the pivot center O1 (best shown in Fig. 2A) of the distal end member 2 relative to the main body marker 7A selected by the actuator shape storage section 53, the information on the shape of the tool 1 with the pivot center O1 taken as a reference selected by the actuator shape storage section 53, and the information on the attitude of the distal end member 2 detected by the tool attitude detector 45.

In other words, the tool processing member position estimator 55 can estimate the absolute position of the pivot center O1 from the information on the position and attitude of the main body marker 7A detected by the marker detecting unit 8, that is, the position and attitude of the reference portion of the actuator main body 10, and the information on the relative position of the pivot center O1 of the distal end member 2 relative to the marker 7A stored in the actuator shape storage section 53. Also, the relative position of the processing member 1a of the tool 1 relative to the pivot center O1 best shown in Fig. 2A can be estimated from the information on the attitude of the distal end member 2 relative to the actuator main body 10, detected by the tool attitude detector 45, and the information on the shape of the tool 1 stored in the actuator shape storage section 53.

From the absolute position of the center of pivot O1 of the distal end member 2 and the relative position of the processing member 1a with the pivot center O1 taken as a reference, so estimated in the manner described above, the absolute position of the processing member 1a of the tool 1 can be estimated. For this reason, relative to the remote controlled actuator 5 capable of altering, by remote control, the attitude of the distal end member 2 for the support of the tool 1, which is provided at the distal end of the spindle guide section 3, the position of the processing member 1a of the tool 1 can be estimated.

The actuator display information generator 55a referred to above and shown in Fig. 4 is operable to calculate an actuator display information, which is information for displaying the position and attitude of the actuator main body 10 and the attitude of the distal end member 2 from various pieces of information used to estimate the position of the tool 1 and then to display a result of such calculation on a screen of the display unit 52. Also, the object display information generator 56 shown in Fig. 4 is operable to calculate an object display information, which is information on the position and attitude of the object marker 7B determined by the marker position and attitude calculator 54B and then to display a result of such calculation on the screen of the display unit 52.

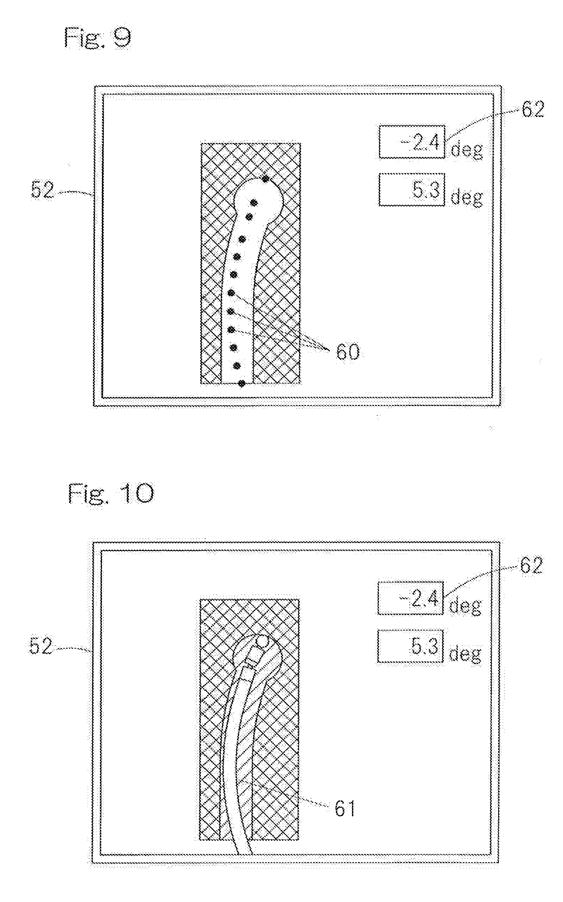

More specifically, as best shown in Fig. 9, the actuator display information and the object display information, both referred to above, that is, the position and attitude of the actuator main body 10, the attitude of the distal end member 2 and the position and attitude of the to-be-processed object 6 are displayed in the form of a plurality of dots 60. Fig. 9 illustrates respective positions of the spindle guide section 3 and the distal end member 2 being displayed in the form of the dots 60 spaced a predetermined distance from each other. Alternatively, as best shown in Fig. 10, using a computer graphics, a representation 61 is displayed, which represents respective contours of the position and attitude of the actuator main body 10, the distal end member 2, the tool 1 and the to-be-processed object 6. Yet, the actuator display information and the object display information may be displayed on display windows 62 in terms of numerical representations together with the dots 60 and the graphic symbol 61 as shown in Figs. 9 and 10. In the illustrated example, there is illustrated a condition in which the attitude of the distal end member 2 is displayed on the display windows 62. It is preferred that information other than the attitude of the distal end member 2 can also be selectively displayed.

Hereinafter, the operation of the remote controlled actuator 5 of the structure shown in and described with particular reference to Fig. 1 will now be described.

When the tool rotation drive source 41 is driven, the rotational force thereof is transmitted to the spindle 13, best shown in Fig. 2A, through the rotary shaft 22, resulting in rotation of the tool 1 together with the spindle 13. By the tool 1 thus driven, cutting of the bone or the like is performed. The rotational speed of the tool 1 can be set to an arbitrary value by means of the rotation operating instrument 50a shown in Fig. 4.

During the procedure, the attitude altering drive source 42 (shown in Fig. 3A) is driven to alter the attitude of the distal end member 2 by remote control. By way of example, if the attitude altering member 31 is advanced by the attitude altering drive source 42 in a direction towards the tip or distal side, the housing 11 for the distal end member 2 is pressed by the attitude altering member 31 with the distal end member 2 consequently altered in attitude along the guide faces F1 and F2 so that the tip or distal side can be oriented downwardly as viewed in Fig. 2A. If the attitude altering member 31 is conversely retracted by the attitude altering drive source 42, the housing 11 for the distal end member 2 is pressed backwardly by the effect of the elastic repulsive force exerted by the restoring elastic member 32 and, consequently, the distal end member 2 is altered in attitude along the guide faces F1 and F2 so that the tip or distal side can be oriented upwardly as viewed in Fig. 2A. At this time, a pressure from the attitude altering member 31, the elastic repulsive force from the restoring elastic member 32 and a reactive force from the constraint member 21 are applied to the distal end member connecting unit 15 and, depending on the balance of those applied forces, the attitude of the distal end member 2 is determined. For this reason, the attitude of the distal end member 2 can be properly controlled by remote control.

Since the attitude altering member 31 is inserted through the guide hole 30a, the attitude altering member 31 can properly act on the distal end member 2 at all times without being accompanied by displacement in position in a direction perpendicular to the lengthwise direction thereof and the attitude altering operation of the distal end member 2 can therefore be performed accurately. Also, since the attitude altering member 31 is comprised of mainly the wire 31a and has a flexible property, the attitude altering operation of the distal end member 2 is carried out accurately even though the spindle guide section 3 is curved. In addition, since the center of the junction between the spindle 13 and the rotary shaft 22 lies at the same position as the respective centers of curvature O1 of the guide faces F1 and F2, no force tending to press and pull will not act on the rotary shaft 22 as a result of the alteration of the attitude of the distal end member 2 and the distal end member 2 can be smoothly altered in attitude.

The remote controlled actuator 5 of the foregoing construction is utilized in grinding the femoral marrow cavity during, for example, the artificial joint replacement surgery and during the surgery, it is used with the distal end member 2 in its entirety or a part thereof inserted into the body of a patient. Because of this, if the distal end member 2 can be altered in attitude by remote control, the bone can be processed in a condition with the tool 1 maintained in a proper attitude at all times and the opening for insertion of the artificial joint can be finished accurately and precisely.

There is the necessity that the rotary shaft 22 and the attitude altering member 31 are provided in a protected fashion. In this respect, the spindle guide section 3, which is elongated in shape, is provided with the rotary shaft 22 at the center of the outer shell pipe 25 and the guide pipe 30, accommodating therein the attitude altering member 31, and the reinforcement shafts 34, all of these are arranged in the circumferential direction and between the outer shell pipe 25 and the rotary shaft 22. Accordingly, the rotary shaft 22 and the attitude altering member 31 can be protected and the interior can be made hollow to thereby reduce the weight without sacrificing the rigidity. Also, the arrangement balance as a whole is rendered good.

Since, as shown in Fig. 2B, the outer diametric surfaces of the rolling bearings 26 supporting the rotary shaft 22 are supported by the guide pipe 30 and the reinforcement shafts 34, the outer diametric surfaces of the rolling bearings 26 can be supported with no need to use any extra member. Also, since the preload is applied to the rolling bearings 26 by means of the spring elements 27A and 27B as shown in Fig. 2A, the rotary shaft 22 comprised of the wire can be rotated at a high speed. Because of that, the processing can be accomplished with the spindle 13 rotated at a high speed and a good finish of the processing can also be obtained and the cutting resistance acting on the tool 1 can be reduced. Since the spring elements 27A and 27B are disposed between the neighboring rolling bearings 26, the spring elements 27A and 27B can be provided with no need to increase the diameter of the spindle guide section 3.

During the operation of the remote controlled actuator 5 shown in Fig. 1, the respective positions of the tool 1 and the to-be-processed object 6 are estimated by the navigation system and are then displayed on the screen of the display unit 52. Because of this, even when the tool 1 is not visible directly with eyes because the tool 1 is then positioned inside the to-be-processed object 6 such as, for example, the bone, the operator can manipulate the tool 1 while looking at the screen of the display unit 52 to ascertain the position of the tool 1 and the position of the to-be-processed object 6. Also, where the respective positions and attitudes of the actuator main body 10, the distal end member 2, the tool 1 and the to-be-processed object 6 are displayed in the form of the plural dots 60 (as shown in Fig. 9) or the contours thereof are displayed in the form of the graphic symbol 61 (as shown in Fig 10), the position of the tool 1 relative to the to-be-processed object 6 can readily be grasped visually.

In the event that the spindle guide section 3 is replaced with a different type and/or the shape of the spindle guide section 3 is deformed, it can be accommodated as the proper relation can be selected by the spindle guide section type selector 53a out from the relations between the types of the spindle guide sections 3 and the relative position of the distal end member 2, which are stored in the actuator shape storage section 53 shown in Fig. 6. Similarly, even in the event that the tool is replaced with a tool of a different shape, it can be accommodated as the proper relation can be selected by the tool type selector 53b from the relations between the types of the tools 1 and the relative position of the processing member 1a, which are stored in the table 53d of the actuator shape storage section 53.

Although the actuator mechanism 5a of the remote controlled actuator 5 shown in Fig. 1 and the navigation computer 9 are positioned having spaced a distance from each other, an electric signal indicative of the attitude of the distal end member 2, detected by the tool attitude detector 45 (best shown in Fig. 3A) provided in the actuator mechanism 5a, can be transmitted to the tool processing member position estimator 55 of the navigation computer 9 through the actuator electric cable 46 and, therefore, transmission of information between the actuator mechanism 5a and the navigation computer 9 can be facilitated.

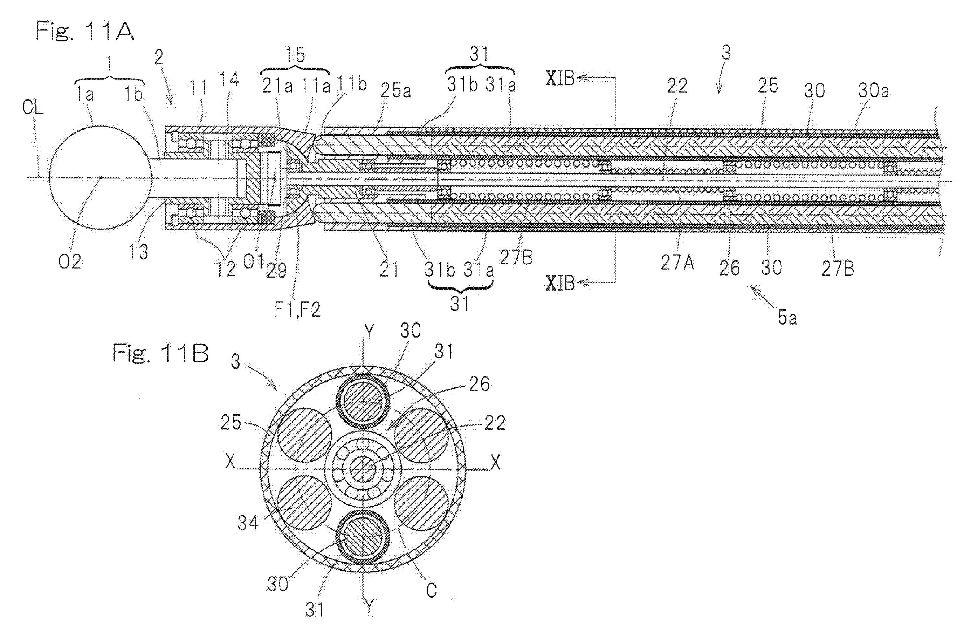

Figs. 11A and 11B illustrate the actuator mechanism 5a of the remote controlled actuator 5 designed in accordance with a second preferred embodiment of the present invention. The spindle guide section 3, which is one of the component parts of the actuator mechanism 5a, is of such a design that as best shown in Fig. 11B, the two guide pipes 30 are provided at the peripheral positions spaced 180° in phase from each other within the outer shell pipe 25 and the attitude altering member 31 is reciprocally movably inserted within guide holes 30a, which are inner diametric holes of the guide pipes 30. As shown in Fig. 11B, between those two guide pipes 30, a plurality of reinforcement shafts 34 are arranged on the same pitch circle as that of the guide pipes 30. As shown in Fig. 11A, no restoring elastic member 32 (such as best shown in Fig. 3A) is provided. The guide faces F1 and F2 are spherical surfaces each having the center of curvature lying at the point O or cylindrical surfaces each having a lateral X-axis as a longitudinal axis passing through the point O.

The drive unit (corresponding to "4" in Fig. 3A) is provided with two attitude altering drive sources (corresponding to "42" in Fig. 3A) for selectively advancing and retracting respective attitude altering members 31 so that when those two attitude altering drive sources 42 are driven in respective directions opposite to each other, the distal end member 2 can be altered in attitude.

By way of example, when the upper attitude altering member 31 shown in Fig. 11A is advanced towards the tip end side and the lower attitude altering member 31 is retracted, the housing 11 for the distal end member 2 is pressed by the upper attitude altering member 31 and, therefore, the distal end member 2 is altered in attitude along the guide surfaces F1 and F2 with the tip end side oriented downwards as viewed in Fig. 11A. Conversely, when both of the attitude altering members 31 are driven in the directions opposite thereto, the lower attitude altering member 31 urges the housing 11 for the distal end member 2 to allow the distal end member 2 to alter in attitude along the guide surfaces F1 and F2 with the distal end side oriented upwardly as viewed in Fig. 11A. At this time, the pressures from the upper and lower attitude altering members 31 and a reactive force from the constraint member 21 act on the distal end member connecting unit 15 and, accordingly, the attitude of the distal end member 2 is determined in dependence on the balance of those working forces.

According to this construction, since the housing 11 for the distal end member 2 is pressed by the two attitude altering members 31, as compared with the previously described embodiment in which it is pressed by the only attitude altering member 31, the attitude stability of the distal end member 2 can be increased.

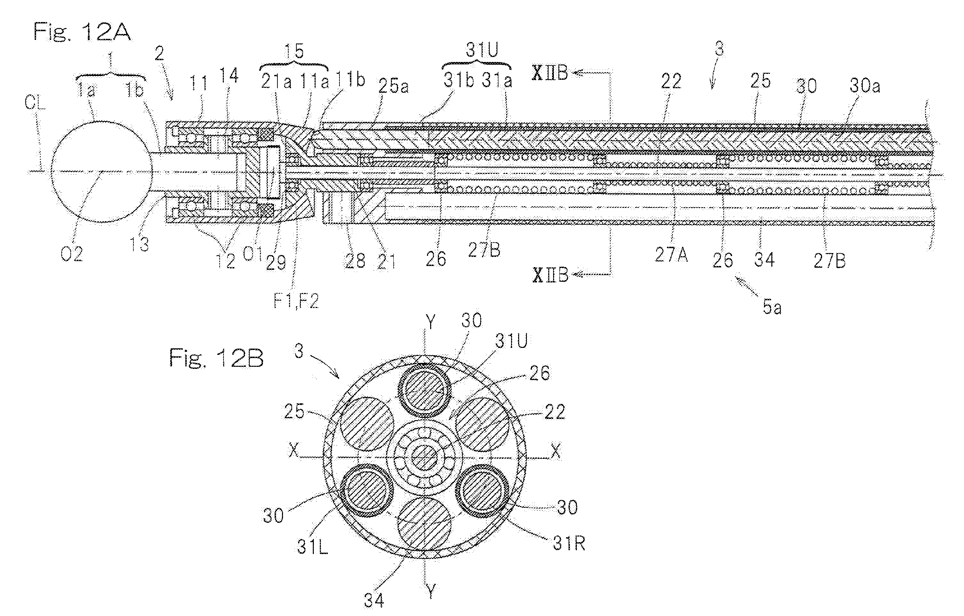

Figs. 12A and 12B illustrate the actuator mechanism 5a employed in the remote controlled actuator 5 designed in accordance with a third preferred embodiment of the present invention. The spindle guide section 3, forming one of the components of the actuator mechanism 5a, is of such a design that as best shown in Fig. 12B, three guide pipes 30 are disposed within the outer shell pipe 25 and positioned at respective circumferential position spaced 120° in phase from each other within the outer shell pipe 25 and, correspondingly, three attitude altering members 31 are accommodated within respective guide holes 30a, which are inner diametric holes of those guide pipes 30, for reciprocal movement relative to the associated guide pipes 30. Between the three guide pipes 30, a plurality of reinforcement shafts 34 are arranged on the same pitch circle as that of the guide pipes 30. As shown in Fig. 12A, no restoring elastic member 32 is provided. The guide surfaces F1 and F2 represents spherical surface having respective centers of curvature lying at the point O and the distal end member 2 can be tilted in any desired direction.

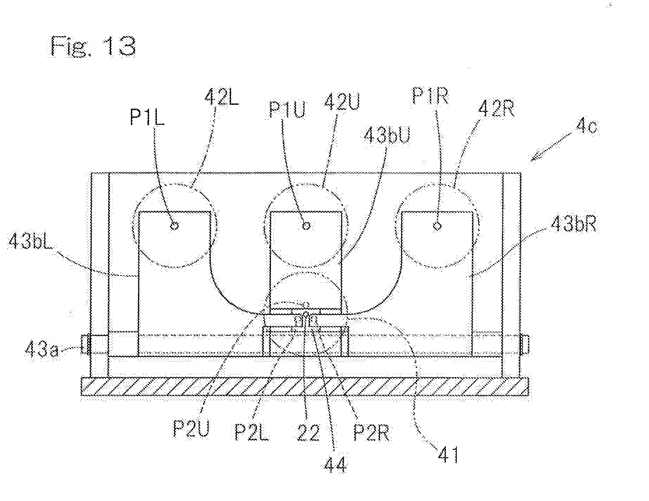

The drive unit is provided with three attitude altering drive sources 42 (42U, 42L and 42R), best shown in Fig. 13, for reciprocally operating respective attitude altering members 31 (31U, 31L and 31R), best shown in Fig. 12B, and those attitude altering drive sources 42 cooperate with each other to drive the distal end member 2 to alter the attitude thereof.

By way of example, when one of the attitude altering members 31U, which is shown in an upper side of Fig. 12A, is advanced towards the tip end side while the other two attitude altering members 31L and 31R are retracted, the housing 11 for the distal end member 2 is pressed by the attitude altering member 31U shown in the upper side of Figs. 12A and 12B to allow the distal end member 2 to be altered in attitude along the guide surfaces F1 and F2 with the tip end side consequently oriented downwardly as viewed in Fig. 12A. At this time, those attitude altering drive sources 42 are controlled so that the amount of advance or retraction of each of the attitude altering members 31 may become proper. On the other hand, when each of those attitude altering members 31 is conversely retracted or advanced, the housing 11 for the distal end member 2 is pressed by the attitude altering members 31L and 31R, which are shown on lower left and lower right sides, and, consequently, the distal end member 2 is altered in attitude along the guide surfaces F1 and F2 with the tip end side oriented upwardly as viewed in Fig. 12A.

Also, when while the attitude altering member 31U on the upper side as viewed in Fig. 12B is held still, the attitude altering member 31L on the left side is advanced towards the tip end side and the attitude altering member 31R on the right side is retracted, the housing 11 for the distal end member 2, best shown in Fig. 12A, is pressed by the attitude altering member 31L on the left side to allow the distal end member 2 to be oriented rightwards, that is, to be altered in attitude along the guide surfaces F1 and F2 with the distal end member 2 oriented towards a rear side of the sheet of the drawing of Fig. 12A. Conversely, when the attitude altering members 31L and 31R on the left and right sides as viewed in Fig. 12B are advanced and retracted, the housing 11 for the distal end member 2, best shown in Fig. 12A, is pressed by the attitude altering member 31R on the right side, allowing the distal end member 2 to be altered in attitude so that the distal end member 2 can be guided along the guide surfaces F1 and F2 so as to be oriented leftwards.

The use of the attitude altering members 31 at the three circumferential locations as hereinabove described is effective to allow the distal end member 2 to be altered in attitude in two axis directions (X-axis and Y-axis directions) upwardly or downwardly and leftwards or rightwards. At this time, respective pressures from the three attitude altering members 31 and the reactive force from the constraint member 21 act on the distal end member connecting unit 15 and, therefore, the attitude of the distal end member 2 is determined in dependence on the balance of those working forces. According to the above described construction, since the housing 11 for the distal end member 2 is pressed by the three attitude altering members 31, the attitude stability of the distal end member 2 can be further increased. It is, however, to be noted that if the number of the attitude altering members 31 used is increased, the attitude stability of the distal end member 2 can be still further increased.

In the case where the distal end member 2 can be altered in attitude in the two axis directions, the attitude of the distal end member 2 in those two axis directions can be detected by detecting the amount of actuation of at least two of the three attitude altering drive sources 42 (Fig. 13) through corresponding actuation amount detectors (not shown). In such case, an aggregation of the actuation amount detectors will form the tool attitude detector 45.

For example, the attitude altering drive mechanism 4c for driving the three attitude altering members 31 is constructed as shown in Fig. 13. In other words, the attitude altering drive mechanism 4c is so constructed that the three attitude altering drive sources 42 (42U, 42L and 42R) for selectively advancing and retracting the attitude altering members 31 (31U, 31L and 31R) may be arranged along a leftward and rightward direction and parallel to each other. Levers 43b (43bU, 43bL and 43bR) corresponding to the attitude altering drive sources 42 may be provided for pivotal movement about a common support pin 43a to enable the force of the output rod 42a (42aU, 42aL and 42aR) of each of the attitude altering drive sources 42 to work on the point P1 (P1U, P1L and P1R) of the respective lever 43b, which is spaced a long distance from the support pin 43a, and to enable the force to work on the attitude altering member 31 at the point P2 (P2U, P2L and P2R), which is spaced a short distance from the support pin 43a. Accordingly, the output of each of the attitude altering drive sources 42 can be increased and then transmitted to the corresponding attitude altering member 31. It is to be noted that the rotary shaft 22 is passed through an opening 44 defined in the lever 43bU for the attitude altering member 31U on the upper side.

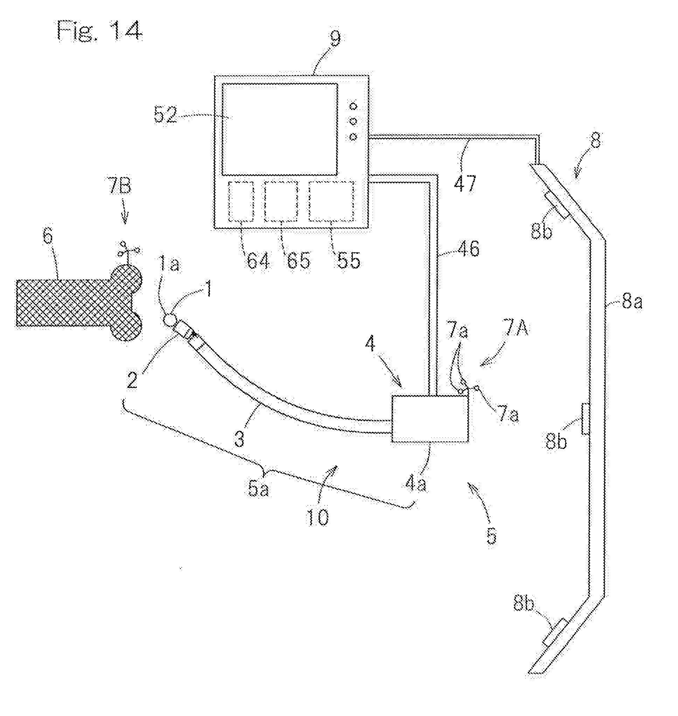

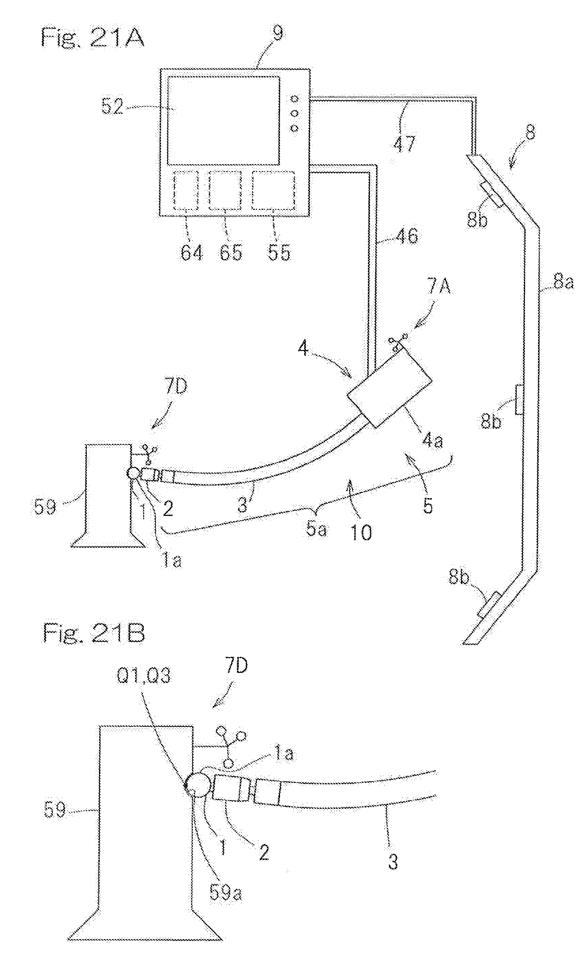

A first mode of application of the present invention will be hereinafter described in detail with particular reference to Figs. 14 to 21A and 21B. In this mode of application, component parts identical or corresponding to those shown and described in connection with the previously described first embodiment of the present invention will be designated by like reference numerals and, therefore, the details thereof are not reiterated for the sake of brevity.

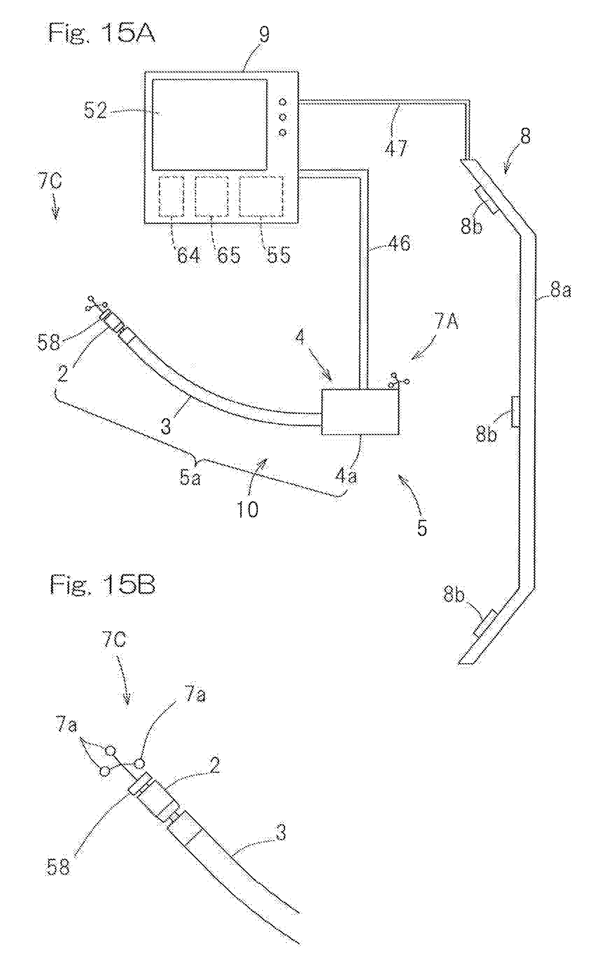

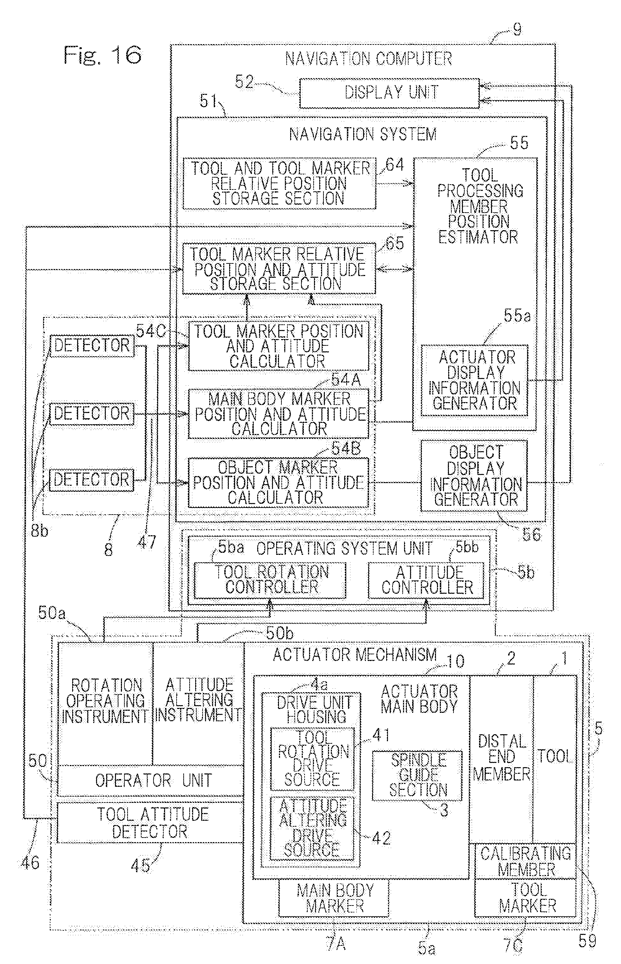

Figs. 14 and Figs. 15A and 15B are diagrams showing a schematic structure of a navigation system for a remote controlled actuator according to the mode of application of the present invention. In particular, Fig. 14 illustrates a condition during which the processing takes place and Figs. 15A and 15B illustrate a condition during which a test is conducted prior to the processing. This mode of application differs from any one of the previously described first to third embodiments in that in the first mode of application, the actuator shape storage section 53 is not employed and a tool marker 7C, as will be described later, is employed together with the markers 7A and 7B and, also, the use is made of a tool marker position and attitude calculator 54C (shown in Fig. 16) with addition of a tool and tool marker relative position storage section 64 and a tool marker relative position and attitude storage section 65. Other structural features are similar to those shown and described in connection with the previously described first embodiment and, therefore, component parts identical or similar to those shown and described in connection therewith are designated by like reference numerals and the details thereof are therefore not reiterated of the sake of brevity.

The navigation system shown in Fig. 14 is of a type employed to the remote controlled actuator 5 capable of controlling the rotation and the attitude of the tool 1 by remote control and includes a marker detecting unit 8 for detecting the position and the attitude of the markers 7A and 7B, fitted respectively to the remote controlled actuator 5 and the to-be-processed object 6, and a marker 7C fitted to a marker carrier member 58 of Fig. 15A mounted on the distal end member 2 in place of the tool 1.

The marker detecting unit 8 makes use of markers 7A, 7B and 7C as respective objects to be detected and includes marker detectors 8b, which are supported by a detector support body 8a, and marker position and attitude calculators 54 built in the navigation computer 9. The main body marker 7A is fitted to the drive unit housing 4a forming a part of the actuator main body 10 and the object marker 7B (Fig. 14) is fitted to the object to be processed 6 such as, for example, a patient's bone or the like. On the other hand, the manner of fitting the tool marker 7C will be described later. In association with the respective marker detectors 8b in the marker detecting unit 8, the actuator, object and tool markers 7A, 7B and 7C are provided with respective sets of three light reflectors 7a. Those three light reflectors 7a are differentiated in position.

Each of the marker detectors 8b is of an optical type and is so designed and so configured as to project a detection beams towards the light reflectors 7a of each of the markers 7A, 7B and 7C and then receive rays of light reflected from those light reflectors 7a. Respective detection signals of those marker detectors 8b are supplied to respective marker position and attitude calculators 54 in the navigation computer 9 through a wiring system (not shown), built in the detector support body 8a, and a marker detector electric cables 47. It is, however, to be noted that the use of three light projectors (not shown) may be provided respectively in the markers 7A, 7B and 7C so that detection beams projected from those light projectors can be received by the individual detectors 8b. The use of the marker detectors 8b of an optical type as discussed above is effective to allow the marker detecting unit 8 to be assembled portable. It is, however, also to be noted that each of the marker detectors 8b may not necessarily be of an optical type and may be of, for example, a magnetic type. The marker detector 8b is the same as that of the first embodiment.

The navigation system unit 51 shown in Fig. 16 includes marker position and attitude calculators 54A, 54B and 54C for the main body, the object to be processed and the tool, respectively, a tool and tool marker relative position storage section 64, a tool marker relative position and attitude storage section 65, a tool processing member position estimator 55. The tool processing member position estimator 56 includes an actuator display information generator 55a. Also, separate from those, the navigation system unit 51 includes an object display information generator 56.

The marker position and attitude calculators 54A, 54B and 54C calculate respective positions and attitudes of the actuator, object and tool markers 7A, 7B and 7C in reference to associated detection signals from the three individual detectors 8b of the marker detecting unit 8. When each of the light reflector 7a of the main body, object and tool markers 7A, 7B and 7C and the individual detectors 8b is employed in three or more in number, the three dimensional position and attitude of each of the markers 7A, 7B and 7C can be determined. The position and attitude of the main body marker 7A are synonymous to the position and attitude of the actuator main body 10. The position and attitude of the object marker 7B are synonymous to the position and attitude of the to-be-processed object 6.

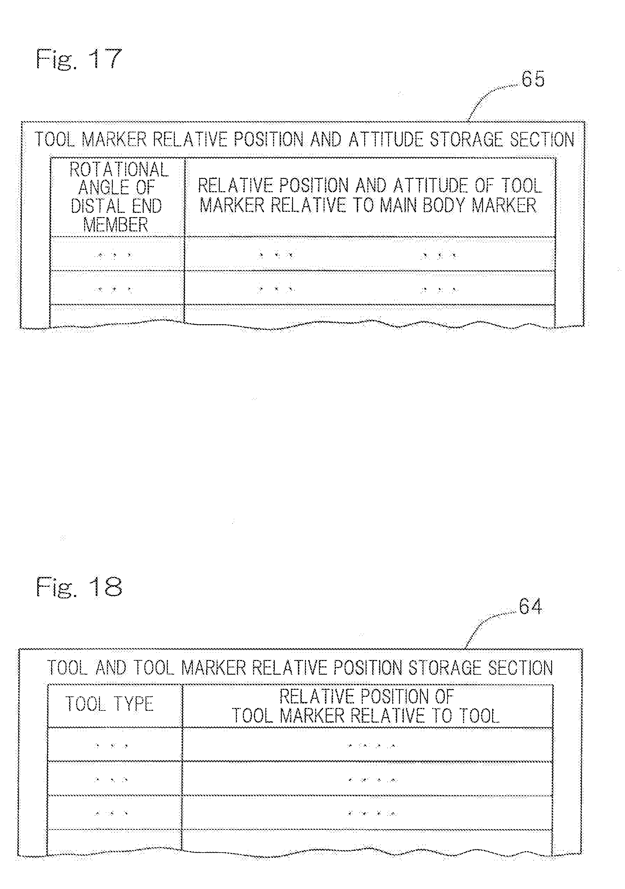

Referring to Fig. 17, the tool marker relative position and attitude storage section 65 is of a kind, in which the relative position and attitude of the tool marker 7C relative to the main body marker 7A are recorded for each angle of rotation of the distal end member 2 detected by the tool attitude detector 45 (Figs. 3A and 16). The relative position and attitude of the tool marker 7C relative to the main body marker 7A are calculated from the position and attitude of the tool marker 7C and the position and attitude of the main body marker 7A, which are detected by the marker detectors 8.

The angle of rotation of the distal end member 2 recorded in the tool marker relative position and attitude storage section 65 and the relative position and attitude of the tool marker 7C relative to the main body marker 7A are obtained by means of an experiment conducted with the use of the actuator mechanism 5a that is actually used in processing. More specifically, the experiment is conducted in a manner as shown in Figs. 15A and 15B. In other words, the tool 1 is removed from the distal end member 2 and, instead of the tool 1, the tool marker 7C is fitted to the distal end member 2 through the maker carrier member 58. In this condition, the attitude altering instrument 50b is operated to alter the attitude of the distal end member 2 relative to the actuator main body 10, and the angle of rotation of the distal end member 2 at that time is detected by the tool attitude detector 45, shown in Fig. 16, and, at the same time, respective positions and attitudes of the main body marker 7A and the tool marker 7C at that time are detected by the marker detecting unit 8. Then, from the position and attitude of the main body marker 7A and the position and attitude of the tool marker 7C, the relative position and attitude of the tool marker 7C relative to the main body marker 7A are calculated. This series of work is performed an arbitrarily chosen number of times with the attitude of the distal end member 2 altered, and for each angle of rotation of the distal end member 2, the relative position and attitude of the tool marker 7C relative to the main body marker 7A are recorded in the tool marker relative position and attitude storage section 65.

The relationship between the position and attitude of the main body marker 7A and the position and attitude of the tool marker 7C depends on the shape of the spindle guide section 3. In a similar fashion to the first embodiment, for example, it differs depending on whether the spindle guide section 3 employed has a curved shape as shown in Fig. 5A or whether it has a straight shape as shown in Fig. 5B. Even where the spindle guide section 3 is, for example, artificially deformed, that before the deformation and that after the deformation differs from each other. Similarly, the above described relation differs depending on the kind of the tool 1 used. For example, it differs depending on whether the processing member 1a of the tool 1 has a spherical shape as shown in Fig. 7A or whether it has a pillar shaped shape as shown in Fig. 7B. For these reasons, when one or both of the spindle guide section 3 and the tool 1 is/are replaced, or when the spindle guide section 3 is deformed, the series of experiments referred to have to be executed in a manner similar to that described hereinabove so as to determine the relative position and attitude of the tool marker 7C relative to the main body marker 7A shown in Fig. 2A.

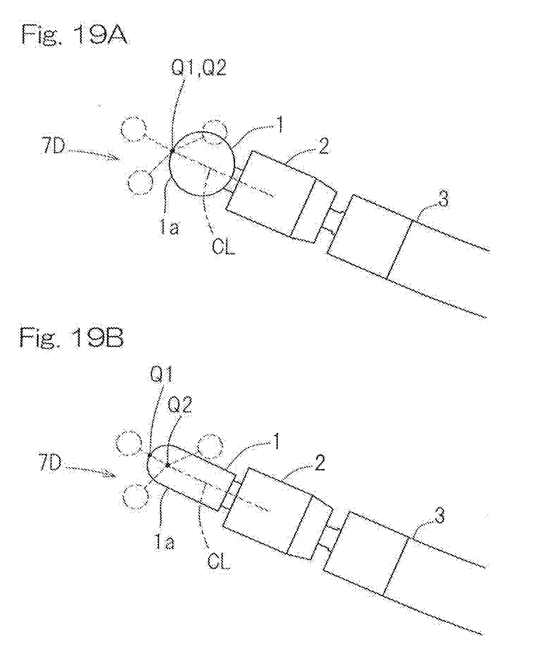

The tool and tool marker relative position storage section 64 stores the relative position of the processing member 1a of the tool 1, which is mounted on the distal end member 2, relative to the tool marker 7C for each type of the tool 1 as shown in Fig. 18. More specifically, depending on whether the tool 1 is of a spherical configuration as shown in Fig. 19A or whether the tool 1 is of a pillar shaped configuration as shown in Fig. 19B, the relative positional relation between the reference point Q1 of the tool 1 and the reference point Q2 of the tool marker 7C varies. This difference in relative positional relation is stored for each type of the tool 1. By way of example, the reference point Q1 of the processing member 1a is rendered to be a point of intersection of an outer peripheral surface of the processing member 1a with the rotational center line CL.

The tool processing member position estimator 55 referred to above and shown in Fig. 16 estimates the absolute position and attitude of the tool marker 7C in reference to the position and attitude of the main body marker 7A, detected by the marker detecting unit 8, and the relative position of the tool marker 7C relative to the main body marker 7A, which are obtained as a result that the angle of rotation of the distal end member 2, detected by the tool attitude detector 45, are checked by the tool marker relative position and attitude storage section 65 and, at the same time, estimates the position of the processing member 1a of the tool 1 by checking a result of such estimation with stored information of the tool and tool marker relative position storage section 54.

In other words, by detecting the angle of rotation of the distal end member 2 with the tool attitude detector 45 and then by checking the detected angle of rotation with the tool marker relative position and attitude storage section 65, the relative position of the tool marker 7C relative to the main body marker 7A is determined. At this time, the actual measurement of the angle of rotation of the distal end member 2 detected by the tool attitude detector 45 does not match with the angle of rotation of the distal end member 2 recorded in the tool marker relative position and attitude storage section 65, but the relative position of the tool marker 7C relative to the main body marker 7A may be determined using the most approximate value or by calculation based on the relative position of the tool marker 7C relative to the main body marker 7A that is delivered from a plurality of values approximate to the actual measurement. Then, when the above described relative position so determined as above is checked with the position and attitude of the main body marker 7A detected by the marker detecting unit 8, the absolute position and attitude of the tool marker 7C can be estimated.

Since the reference point Q1 of the processing member 1a of the tool 1 in a condition mounted on the distal end member 2 relative to the reference point Q2 of the tool marker 7C shown in Figs. 19A and 19B lies at a predetermined relative position, the position of the processing member 1a of the tool 1 can be ascertained if the absolute position and attitude of the tool marker 7C shown in Figs. 15A and 15B is obtained.

The operation of the remote controlled actuator 5 shown in Fig. 14 will now be described in detail.

During the processing, the marker carrier member 58 equipped with the tool marker 7C shown in Fig. 15A is removed from the distal end member 2 and the tool 1 shown in Fig. 14 is then mounted. If, in this condition, the tool rotation drive source 41 is driven, the rotational force thereof is transmitted to the spindle 13 through the rotary shaft 22 and the spindle 13 is then rotated together with the tool 1. By the tool 1 then rotating, cutting of the bone or the like is performed. The rotational speed of the tool 1 can be set arbitrarily by means of the rotation operating instrument 50a shown in Fig. 16. Other functional features than that described above are similar to those shown and described in connection with the previously described first embodiment and, therefore, the further details thereof are not reiterated for the sake of brevity.

In the event that the tool 1 and/or the spindle guide section 3 are replaced, the experiment shown in Figs. 15A and 15B is carried out with the use of the actuator mechanism 5a after the replacement and the recorded contents of the tool marker relative position and attitude storage section 65 are rewritten. By so doing, it can accommodate the replacement of the tool 1 and/or the spindle guide section 3.

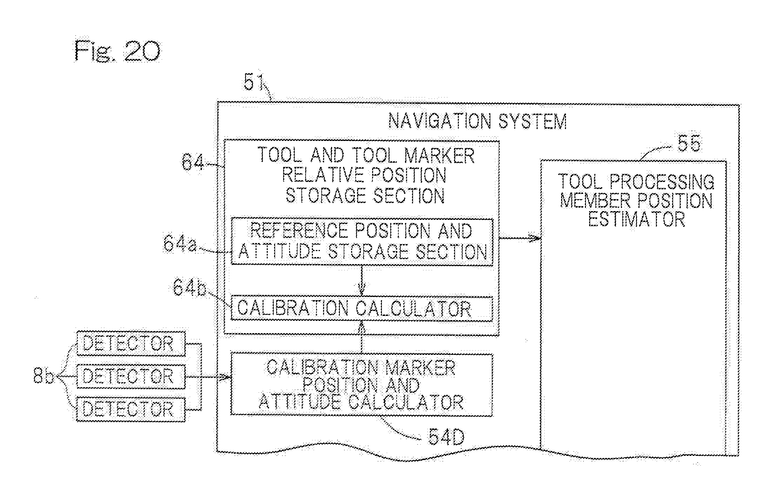

Fig. 20 illustrates a different structure of the tool and tool marker relative position storage section 65. This tool and tool marker relative position storage section 64 is not of a design storing the information on the relative position of the tool marker 7C relative to the tool 1 beforehand for each type of the tool 1 as is the case with that in the previously described embodiment, but is of a design in which only information on the tool 1 which forms a reference is stored in a reference position and attitude storage unit 64a, but in which based on a calibration work shown in Figs. 21A and 21B, the information on the various types of the tools 1 is calibrated by a calibration calculator 64b shown in Fig. 20.

More specifically, using a calibrating member 59 fitted to a calibration marker 7D, the previously described calibrating work is performed. In other words, the tool 1, which defines the reference, is mounted on the distal end member 2 and, in a condition in which the reference point Q1 of the processing member 1a of the tool 1 is held in contact with a predetermined calibrating point Q3 of the calibrating member 59, the position and attitude of the calibration marker 7D are detected by the marker detecting unit 8. A result of this detection is stored in the reference position and attitude storage unit 64a, shown in Fig. 20, as a reference value of the position and attitude of the calibrating marker 7D.

Where the tool 1 other than the tool 1 defining the reference is used, that tool 1 is mounted on the distal end member 2 and, in a condition in which the reference point Q1 of the processing member 1a of the tool 1 is held in contact with the calibrating point Q3 of the calibrating member 59, the position and attitude of the calibration marker 7D are detected by the marker detecting unit 8. The calibration calculator 64b shown in Fig. 20 compares a result of this detection with the reference value, recorded in the reference position and attitude storage unit 64a and calibrates the relative position of the processing member 1a of the tool 1 in the condition as mounted on the distal end member 2 relative to the tool marker 7C.

According to the foregoing construction, if only reference values of the position and attitude of the calibration marker 7D are stored, it can accommodate the tool 1 of a different type merely by means of a simple operation in which in the condition with the reference point Q1 of the processing member 1a of the tool 1 held in contact with the calibrating point Q3 of the calibrating member 59, the position and attitude of the calibration marker 7D are detected by the marker detecting unit 8. For this reason, there is no need to conduct the experiment of detecting the relative position of the processing member 1a of the tool in the condition as mounted on the distal end member 2 relative to the tool marker 7C for each type of the tool 1.

An outer surface shape of the calibrating member 59 in proximity to the calibration point Q3 represents a concave shape defined by a curved surface 59a following a curved outer surface of the processing member 1a of the tool 1. In the case of this mode of application, the curved surface 59a is a spherical surface. For this reason, when the reference point Q1 of the tool 1 is to be held in contact with the calibration point Q3 of the calibrating member 59, the reference point Q1 of the tool 1 can be held in contact with the calibration point Q3 of the calibrating component 59 with a high accuracy and the calibrating accuracy of the relative position of the processing member 1a of the tool 1 in the condition as mounted on the distal end member 2 relative to the tool marker 7C can be increased.

The mode of application hereinabove described includes the following applied modes, in which as compared with any one of the previously described first to third preferred embodiments, the actuator shape storage section 53 is omitted, the tool marker 7C and the concomitant tool marker position and attitude calculator 54C are added, and the tool and tool marker relative position storage section 64 and the tool marker relative position and attitude storage section 65 are added as requirements.

### [Mode 1]

The navigation system for the remote controlled actuator according to a mode 1 is a navigation system for estimating the position of the processing member 1a of the tool 1, which is applicable to the remote controlled actuator 5 including an actuator main body 10 in which a base end of the spindle guide section 3 of the elongated configuration is coupled with the drive unit housing 4a; a distal end member 2 fitted to a distal end of the spindle guide section 3 for pivotal movement about a pivot center O for altering the attitude thereof; a tool 1 rotatably supported by the distal end member 2; an attitude altering drive source 42 and a tool rotation drive source 41 provided within the drive unit housing 4a for effecting an alternation in attitude of the distal end member 2 and a rotation of the tool 1, respectively; and an operator unit 50, provided within the drive unit housing 4a, for controlling respective operation of the drive sources 42 and 41 to performing an operation of the attitude of the distal end member 2 and of the rotation of the tool 1.

The navigation system includes a marker detecting unit 8 for detecting respective positions and attitudes of the main body marker 7A, fitted to the drive unit housing 4a of the actuator main body 10, and the tool marker 7C fitted to a marker carrier member 58, removably mounted on the distal end member 2 in place of the tool 1, and positioned at a predetermined relative position relative to the tool 1 in a condition as mounted on the distal end member 2; the tool and tool marker relative position storage section 64 for storing the relative position of a processing member 1a of the tool 1 in a condition as mounted on the distal end member 2 relative to the tool marker 7C; the tool attitude detector 45 for detecting the angle of pivot of the distal end member 2 about the pivot center O relative to the actuator main body 10; the tool marker relative position and attitude storage section 65 in which for each angle of pivot of the distal end member 2 detected by the tool attitude detector 45, the relative position and attitude of the tool marker 7C in the condition as mounted on the distal end member 2 relative to the main body marker 7A, which have been calculated from the position and attitude of the tool marker 7C and the position and attitude of the main body marker 7A detected by the marker detecting unit 8, are recorded.

The navigation system further includes the tool processing member position estimator 55 for estimating the position of the processing member 1a of the tool 1 by estimating the absolute position and attitude of the tool marker 7C in the condition as fitted to the distal end member 2 through the marker carrier member 58 from the relative position of the tool marker 7C in the condition as mounted on the distal end member 2 relative to the main body marker 7A, which is obtained by checking the position and attitude of the main body maker 7A, detected by the marker detecting unit 8, and the angle of pivot of the distal end member 2, detected by the tool attitude detector 45, by means of the tool marker relative position and attitude storage section 65 and also by checking a result of that estimation and the stored information of the tool and tool marker relative position storage section 64.

### [Mode 2]

The marker carrier member 58 is mounted on the distal end member 2 in place of the tool 1 during the experiment that is conducted prior to the actual processing. The tool marker relative position and attitude storage section 65 is of a type in which, for example, the relative position and attitude of the tool marker 7C in the condition as mounted on the distal end member 2 relative to the main body marker 7A, which are obtained as a result of that experiment, are recorded.

The navigation system of the above described construction performs the following experiment with the use of the remote controlled actuator 5, which is actually used in processing, prior to the processing. In other words, the tool 1 is removed from the distal end member 2 and, instead of the tool 1, the tool marker 7C is fitted to the distal end member 2 through the marker carrier member 58. The relative position of the processing member 1a of the tool 1 in the condition as mounted on the distal end member 2 relative to the tool marker 7C is stored in the tool and tool marker relative position storage section 64. In this condition, the attitude of the distal end member 2 is altered relative to the actuator main body 10 by manipulating the operator unit 50 and the angle of pivot of the distal end member 2 at that time is detected by the tool attitude detector 45 and, at the same time, the respective positions and attitudes of the main body marker 7A and the tool marker 7C at that time are detected by the marker detecting unit 8. Then, from the position and attitude of the main body marker 7A and the position and attitude of the tool marker 7C, the relative position and attitude of the tool marker 7C relative to the main body marker 7A are calculated. This series of works are performed an arbitrarily chosen number of time with the attitude of the distal end member 2 altered and for each angle of pivot of the distal end member 2, the relative position and attitude of the tool marker 7C relative to the main body marker 7A are recorded in the tool marker relative position and attitude storage section 65.

During the processing, the marker carrier member 58 and the tool marker 7C are removed from the distal end member 2 and the tool 1 is then mounted. The angle of pivot of the distal end member 2, detected by the tool attitude detector 45, and the position and attitude of the main body marker 7A, detected by the marker detecting unit 8, are inputted to the tool processing member position estimator 55. Those pieces of information change from time to time as the remote controlled actuator 5 is operated. In the tool processing member position estimator 55, from the position and attitude of the main body marker 7A and the relative position of the tool marker 7C in the condition as mounted on the distal end member 2 relative to the main body marker 7A, which is obtained by checking the position and attitude of the main body marker 7A and the angle of pivot of the distal end member 2 by means of the tool marker relative position and attitude storage section 65, the absolute position and attitude of the tool marker 7C in the condition as mounted on the distal end member 2 at that time are estimated. Also, the position of the processing member 1a of the tool 1 is estimated by checking a result of that estimation and the recorded information of the tool and tool marker relative position storage section 64. By so doing, relative to the remote controlled actuator 5 capable of altering the attitude of the distal end member 2 for the support of the tool, provided at the distal end of the spindle guide section 3, by remote control, the position of the processing member 1a of the tool 1 during the processing can be estimated.

### [Mode 3]

In the above described mode 1, included is the calibrating member 59 having the calibration marker 7D fitted thereto, and the tool and tool marker relative storage section 64 may include the reference position and attitude storage unit 64a and a calibration calculator 64b. The reference position and attitude storage unit 64a records the reference value of the position and attitude of the calibration marker 7D, detected by the marker detecting unit 8, in a condition in which the tool 1 defining the reference is mounted on the distal end member 2 and the reference point Q1 of the processing member 1a of the tool 1 is held in contact with the predetermined calibration point Q3 of the calibrating member 59. The calibration calculator 64b calibrates the relative position of the processing member 1a of the tool 1 in the condition as mounted on the distal end member 2 relative to the tool marker 7C by comparing the position and attitude of the calibration marker 7D, detected by the marker detecting unit 8, with the reference value, recorded in the reference position and attitude storage unit 64a, in the condition in which the tool 1 used in processing is mounted on the distal end member 2 and the reference point Q1 of the processing member 1a of the tool 1 is held in contact with the calibration point Q3 of the calibrating member 59.

According to the above described construction, in the condition, in which the tool 1 defining the reference is mounted on the distal end member 2 and the reference point Q1 of the processing member 1a of the tool 1 is held in contact with the predetermined calibration point Q3 of the calibrating member 59, the position and attitude of the calibration marker 7D are detected by the marker detecting unit 8. A result of this detection is stored in the reference position and attitude storage unit 64a as the reference values of the position and attitude of the calibration marker 7D. Where the tool 1 other than the tool 1 defining the reference is used, in the condition, in which that tool 1 is mounted on the distal end member 2 and the reference point Q1 of the processing member 1a of that tool 1 is held in contact with the calibration point Q3 of the calibrating member 59, the position and attitude of the calibration marker 7D are detected by the marker detecting unit 8. The calibration calculator 64b compares the result of this detection with the reference values recorded in the reference position and attitude storage unit 64a to calibrate the relative position of the processing member 1a of the tool 1 in the condition as mounted on the distal end member 2 relative to the tool marker 7C.

The relative position of the processing member 1a of the tool 1 in the condition as mounted on the distal end member 2 relative to the tool marker 7C varies depending on the type and model of the tool 1 used. For this reason, it is generally required that for each type of the tool 1, the relative position referred to above is determined by conducting a series of experiments and is then stored in the tool and tool marker relative position storage section 64. However, by the provision of the reference position and attitude storage unit 64a and the calibration calculator 64b, if only the reference values of the position and attitude of the calibration marker 7D are stored, it can accommodate a different type of the tool 1 merely by a simplified procedure of detecting the position and attitude of the calibration marker 7D by means of the marker detecting unit 8 in the condition in which the reference point Q1 of the processing member 1a of the tool 1 is held in contact with the calibration point Q3 of the calibrating member 59. For this reason, there is no necessity to conduct the experiments to detect the relative position of the processing member 1a of the tool 1 in the condition as mounted on the distal end member 2 relative to the tool marker 7C for each type of the tool 1.

### [Mode 4]

In the above described mode 3, where the processing member 1a of the tool 1 represents a curved shape, in which at least a portion of an outer surface containing the reference point Q1 is protruding outwardly, the outer surface shape of the calibrating member 59 in proximate to the calibration point Q3 preferably represents a concaved shape defined by a curved surface 59a following the outer surface of a curved surface shape of the processing member 1a of the tool 1.

When the reference point Q1 of the tool 1 is to be contacted with the calibration point Q3 of the calibrating member 59, if the outer surface shape of the calibrating member 59 in the vicinity of the calibration point Q3 is the concaved shape defined by the curved surface 59a following the outer surface of the curved surface shape of the processing member 1a of the tool 1, the reference point Q1 of the tool 1 can be brought into contact with the calibration point Q3 of the calibrating member 59 with high accuracy and, therefore, the calibrating accuracy of the relative position of the processing member 1a of the tool 1 in the condition as mounted on the distal end member 2 relative to the tool marker 7C can be increased.

### [Mode 5]

In the above described mode 1, each of the main body marker and the tool marker is of a type capable of projecting light or reflecting light and the marker detecting unit can be of an optical type capable of receiving light from the main body marker and the tool marker. The marker detecting unit of the optical type has a simplified structure.

### [Mode 6]

In the above described mode 1, each of the main body marker, the tool marker and the calibration marker is of a type capable of projecting light or reflecting light and the marker detecting unit can be of an optical type capable of receiving light from the main body marker, the tool marker and the calibration marker.

### [Mode 7]

In the above described mode 1, the tool attitude detector 45 may be provided in the attitude altering drive source 42 or a drive system for transmitting an operation from the attitude altering drive source 42 to the distal end member 2 and is operable to output an electric signal corresponding to the attitude of the distal end member 2.

### [Mode 8]

In the above described mode 1, a display unit 52 may be provided for displaying images such that the tool processing member position estimator 55 is provided with an actuator display information generator 55a for calculating an actuator display information, which is information for displaying the position and attitude of the actuator main body 10 and the attitude of the distal end member 2, from various input information that is used in the estimation of the position of the tool 1, and then displaying such actuator display information on a screen of the display unit 52.

### [Mode 9]

In the mode 8, where the display unit 52 and the actuator display information generator 55a are employed, the actuator display information generated by the actuator display information generator 55a may be information necessary to display the position and attitude of the actuator main body 10 and the attitude of the distal end member 2 on the screen of the display unit 52 in the form of series of dots 60.

### [Mode 10]

In the above described mode 8, the actuator display information generator 55a may be of a type generating, as the actuator display information, a graphic symbol 61 and then displaying the graphic symbol 61 on the screen of the display unit 52, which symbol 61 is representative of an external shape of the tool 1, the distal end member 2 and the actuator main body 10, which reflect the position and attitude of them, by means of a computer graphics.

### [Mode 11]

In the above described mode 8, the actuator display information generated by the actuator display information generator 55a may be information in the form of a numeral on the screen of the display unit 52.

### [Mode 12]

In the above described mode 1, the remote controlled actuator is preferably constructed as follows. That is to say, the distal end member 2 rotatably supports the spindle 13 for holding the tool 1; the spindle guide section 3 includes a rotatable shaft 22 for transmitting rotation of the tool rotation drive source 41 within the drive unit housing 4a to the spindle 13 and an attitude altering member 31 for altering the attitude of the distal end member 2 by selectively advancing or retracting by means of the attitude altering drive source 42 within the drive unit housing 4a in a condition with the tip end held in contact with the distal end member 2.

Although in describing the navigation system for the remote controlled actuator reference has been made to that for the medical use, the present invention can be equally applied to the navigation system for the remote controlled actuator for use in any application. By way of example, if the remote controlled actuator is used in performing a mechanical processing, a drilling process for drilling a curved hole and a cutting process to be performed at a site deep in the groove can be accomplished.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. Accordingly, such changes and modifications are, unless they depart from the scope of the present invention as delivered from the claims annexed hereto, to be construed as included therein.

### [Reference Numeral]

- 1 ····: Tool
- 1a ····: Processing member
- 2 ····: Distal end member
- 3 ····: Spindle guide section
- 4a ····: Drive unit housing
- 5 ····: Remote controlled actuator
- 6 ····: Object to be processed
- 7A, 7B ····: Marker
- 8 ····: Marker detecting unit
- 9 ····: Navigation computer
- 10 ····: Actuator main body
- 13 ····: Spindle
- 22 ····: Rotary shaft
- 31 ····: Attitude altering member
- 41 ····: Tool rotation drive source
- 42 ····: Attitude altering drive source
- 45 ····: Tool attitude detector
- 50 ····: Operator unit
- 52 ····: Display unit
- 53 ····: Actuator shape storage section
- 53a ····: Spindle guide section type selector
- 53b ····: Tool type selector
- 54A to 54C ····: Marker position and attitude calculator
- 55 ····: Tool processing member position estimator
- 55a ····: Actuator display information generator
- 58 ····: Maker carrier member
- 59 ····: Calibrating member
- 64 ····: Tool and tool marker relative position storage section
- 65 ····: Tool marker relative position and attitude storage section

## Claims

1. A navigation system for estimating position of a tool relative to a remote controlled actuator, the actuator including an actuator main body including a spindle guide section of an elongated configuration having a base end connected with a drive unit housing; a distal end member fitted to a distal end of the spindle guide section for pivotal movement about a pivot center to alter in attitude thereof; the tool rotatably being supported by the distal end member; an attitude altering drive source and a tool rotation drive source provided within the drive unit housing for altering an attitude of the distal end member and for rotating the tool, respectively; and an operator unit provided within the drive unit housing for controlling respective operations of the drive sources to operate the attitude of the distal end member and the rotation of the tool, the navigation system comprising:
a marker detecting unit for detecting a position and an attitude of a marker fitted to the drive unit housing of the actuator body;
a tool attitude detector for detecting the attitude of the distal end member relative to the actuator body;
an actuator shape storage section for storing information on a relative position of the pivot center relative to the marker and information on the shape of the tool with reference to the pivot center; and
a tool processing member position estimator for estimating a position of a processing member of the tool from information on the position and attitude of the marker detected by the marker detecting unit; the information on the relative position of the pivot center and the information on the shape of the tool, both stored in the actuator shape storage section, and information on the attitude of the distal end member detected by the tool attitude detector.

2. The navigation system for the remote controlled actuator as claimed in claim 1,
in which the navigation system is useable in a plurality of remote controlled actuators utilizing respective different types of spindle guide sections, and
in which the actuator shape storage section stores the information on the relative position of the pivot center relative to the marker for each type of the spindle guide sections, and
further comprising a spindle guide section type selector for selecting the information on the relative position of the pivot center that is to be used in estimation performed by the tool processing member position estimator.

3. The navigation system for the remote controlled actuator as claimed in claim 1,
in which the navigation system is usable in a plurality of remote controlled actuators utilizing respective different types of tools and a remote controlled actuator of a type, in which different types of the tools can be replaced one at a time, wherein the actuator shape storage section stores the information on the shape of the tool for each type of the tool with reference to the pivot center, and
further comprising a tool type selector for selecting the information on the shape of the tool that is to be used in estimation performed by the tool processing member position estimator.

4. The navigation system for the remote controlled actuator as claimed in claim 1, in which the marker is of a type capable of projecting or reflecting light and the marker detecting unit is of an optical type capable of receiving the light from the marker.

5. The navigation system for the remote controlled actuator as claimed in claim 1, in which the tool attitude detector is provided in the attitude altering drive source or a drive system for transmitting an operation from the attitude altering drive source to the distal end member and is operable to output an electric signal corresponding to the attitude of the distal end member.

6. The navigation system for the remote controlled actuator as claimed in claim 1, further comprising a display unit for displaying images and in which the tool processing member position estimator is provided with an actuator display information generator for calculating an actuator display information, which is information for displaying the position and attitude of the actuator main body and the attitude of the distal end member, from various input information that is used in the estimation of the position of the tool, and then displaying such actuator display information on a screen of the display unit.

7. The navigation system for the remote controlled actuator as claimed in claim 6, in which the actuator display information generated by the actuator display information generator is information necessary to display the position and attitude of the actuator main body and the attitude of the distal end member on a screen of the display unit in the form of series of dots.

8. The navigation system for the remote controlled actuator as claimed in claim 6, in which the actuator display information generator is of a type generating, as the actuator display information, a graphic symbol and then displaying the graphic symbol on a screen of the display unit, which symbol is representative of an external shape of the tool, the distal end member and the actuator main body, which reflect the position and attitude of them, by means of a computer graphics.

9. The navigation system for the remote controlled actuator as claimed in claim 6, in which the actuator display information generated by the actuator display information generator is information in the form of a numeral on a screen of the display unit.

10. The navigation system for the remote controlled actuator as claimed in claim 1, in which the distal end member rotatably supports the spindle for holding the tool; the spindle guide section includes a rotatable shaft for transmitting rotation of the tool rotation drive source within the drive unit housing to the spindle and an attitude altering member for altering the attitude of the distal end member by selectively advancing or retracting by means of the attitude altering drive source within the drive unit housing in a condition with the tip end held in contact with the distal end member.
